(19) Europäisches Patentamt
European Patent Office
Office européen des brevets

(11) EP 3 110 783 B1

(12) **FASCICULE DE BREVET EUROPEEN**

(45) Date de publication et mention de la délivrance du brevet:
**08.04.2020 Bulletin 2020/15**

(21) Numéro de dépôt: **15710832.5**

(22) Date de dépôt: **27.02.2015**

(51) Int Cl.:
***C07C 41/30*** *(2006.01)*

(86) Numéro de dépôt international:
**PCT/FR2015/050479**

(87) Numéro de publication internationale:
**WO 2015/128593 (03.09.2015 Gazette 2015/35)**

(54) **PROCEDE DE PREPARATION A HAUTS RENDEMENTS DE P-(R)CALIX[9-20]ARENES**

VERFAHREN ZUR HERSTELLUNG VON P-(R)CALIX[9-20]ARENEN MIT HOHER AUSBEUTE

PROCESS FOR HIGH-YIELD PREPARATION OF P-(R)CALIX[9-20]ARENES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.02.2014 FR 1451660**

(43) Date de publication de la demande:
**04.01.2017 Bulletin 2017/01**

(73) Titulaires:
- **Université Paris Sud XI**
  **91405 Orsay Cedex (FR)**
- **Centre National de la Recherche Scientifique**
  **75794 Paris Cedex 16 (FR)**

(72) Inventeurs:
- **HUC, Vincent, Germain**
  **F-91400 Orsay (FR)**
- **MARTINI, Cyril**
  **F-91140 Villebon sur Yvette (FR)**

(74) Mandataire: **Grosset-Fournier, Chantal Catherine et al**
**Grosset-Fournier & Demachy**
**54, rue Saint-Lazare**
**75009 Paris (FR)**

(56) Documents cités:
**WO-A1-2013/088056**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 110 783 B1

## Description

La présente invention concerne un procédé de préparation à hauts rendements de *p*-(R)calix[9-20] arènes.

Les calixarènes ont été particulièrement étudiés ces trente dernières années en raison des possibilités immenses offertes par ces macrocycles aisément accessibles. Ces macrocycles possèdent une forme de calice dont la cavité est généralement inférieure ou égale à environ 1 nm.

Quelques unes des propriétés les plus étudiées incluent les phénomènes de reconnaissance des ions inorganiques et des molécules organiques, les assemblages supramoléculaires ou bien encore la synthèse des nanoparticules, entre autres.

Dans la plupart des cas, ces études ont été effectuées avec les calixarènes fonctionnalisés par un *p*-(*t*-butyl) ou par des calixarènes obtenus par modification chimique de ces *p*-(*t*-butyl)calixarènes, étant donné que la synthèse des *p*-(*t*-butyl)calixarènes est de loin la plus documentée depuis le travail pionnier de Gutsche et al.D. Gutsche, Calixarenes: An Introduction, The Royal Society of Chemistry, Cambridge, 2008**.** Z. Asfari, V. Böhmer, J. Harrowfield, J. Vicens, Calixarenes 2001, Kluwer, Dordrecht, the Netherlands, 2001**.**

Par conséquent, l'utilisation d'autres monomères de type phénols fonctionnalisés pour la synthèse des calixarènes est en grande partie inexplorée, même si des synthèses en une étape d'autres *p*-(alkyl)calixarènes sont connues (T. Patrick, P. Egan, J. Org. Chem. 1977, 42, 382 ; T. Patrick, P. Egan, J. Org. Chem. 1978, 43, 4280 ; Z. Asfari, J. Vicens, Tetrahedron Lett. 1988, 29, 2659 ; F. Vocanson, M. Perrin, R. Lamartine, J. Inclusion Phenom. Macrocyclic Chem. 2001, 39, 127 ; Jerry L. Atwood et al; Org. Lett. 1999, 1, 1523).

Les calixarènes *p*-substitués sont habituellement obtenus en mélange de *p*-calix[4, 5, 6, 7, 8]arènes par réaction d'un phénol *p*-substitué avec du paraformaldéhyde en présence d'au moins une base telle que la potasse ou la soude (B. Dahwan et al., Macromolec. Chem. 1987, 188, 921 ; C. D. Gutsche et al., Org. Synth. 1990, 68, 234 ; C. D. Gutsche et al., Org. Synth. 1990, 68, 238).

Dans la plupart des cas, le retrait ou la substitution des groupements alkyles en para de la fonction hydroxyle de ces calixarènes, lorsqu'elle est possible, est au mieux délicate. Dans le cas le plus courant des *p*-(*t*-butyl)calixarènes, il s'agit en général d'un procédé en plusieurs étapes. Dans un premier temps, un réactif de type acide de Lewis est généralement associé à un phénol pour retirer le groupement *t*-butyle. Dans un second temps, une autre fonction peut être introduite en lieu et place du groupement *t*-butyle par substitution électrophile. La réaction n'étant pas quantitative, la « déterbutylation » devient problématique, notamment lorsque le nombre d'unités du calixarène augmente. La présence de sous-produits limite le rendement, impose une étape de purification et restreint la pureté du produit. Ceci peut limiter le rendement final en produit entièrement déprotégé ainsi que son utilisation.

Les *p*-(benzyloxy)calixarènes représentent une alternative très intéressante au *p*-(alkyl)calixarènes. Les motifs (benzyloxy)phénols sont en effet quantitativement réduits en phénols par hydrogénolyse catalysée par le palladium sur charbon, autorisant une post-fonctionnalisation aisée.

Peu de documents décrivent la synthèse de grands calixarènes, c'est-à-dire comportant de 9 à 20 motifs de répétition.

Ainsi le brevet CA 2,251,070 décrit l'obtention avec de faibles rendements des calixarènes comportant 9 et 11-14 motifs de répétition avec un procédé en deux étapes par réaction d'un p-(alkyl) ou *p*-(aralkyl)phénol en milieu aqueux en présence d'une base en quantité inférieure à 0,5 eq puis chauffage dans un solvant organique sans eau.

Ce brevet ne décrit pas l'obtention de *p*-(R-oxy)calix[9-20]arènes.

L'article de Gutsche et al. (J. Am. Chem. Soc. 1999, 121, 4136) décrit uniquement l'obtention de *p*-(*t*-butyl)calix[9-20]arènes en milieu acide mais pas, en particulier, l'obtention de *p*-(R-oxy)calixarènes.

La demande internationale WO 2013/088056 divulgue la synthèse de *p*-(benzyloxy)calix[6,7,8]arènes obtenus en mélange avec des *p*-(benzyloxy)calix[9-20] arènes, mais ces derniers sont des sous-produits du procédé mis en œuvre qui sont obtenus en proportion minoritaire et ne sont pas isolés.

Par conséquent, l'obtention de grands calixarènes, qui sont de plus aisément fonctionnalisables sur la couronne haute par une large variété de groupements chimiques dans des conditions douces, est toujours une question ouverte et notamment l'obtention de grands calixarènes tels que les *p*-(R)calix[9-20]arènes, en particulier les *p*-(R-oxy)calix[9-20] arènes, avec de bons rendements.

L'un des objets de l'invention est la fabrication de *p*-(R)calix[9-20]arènes, en particulier de *p*-(R-oxy)calix[9-20] arènes avec des rendements cumulés supérieurs à 5%, notamment supérieurs à 10, 15, 20, 25, 30, 35, 40, 45 ou 50%.

L'un des objets de l'invention est la fabrication de *p*-(R)calix[9-12]arènes, en particulier de *p*-(R-oxy)calix[9-12] arènes avec des rendements cumulés supérieurs à 5%, notamment supérieurs à 10, 15, 20, 25, ou 30%.

Un autre objet de l'invention est de fournir un procédé de synthèse permettant l'obtention d'un mélange de douze *p*-(R)calix[9-20]arènes, en particulier de douze *p*-(R-oxy)calix[9-20]arènes, ou de quelques *p*-(R)calix[9-20]arènes, en particulier de quelques *p*-(R-oxy)calix[9-20]arènes, isolés et purs, ou un mélange de deux, trois, quatre, cinq, six, sept, huit, neuf, dix ou onze parmi les douze, ainsi qu'une procédure de purification permettant de les obtenir purs, en particuliers exempts de *p*-(R)calixarènes géants, dont la taille est supérieure à 20, c'est-à-dire des *p*-(R)calixarènes

comportant plus de 20 motifs de répétition.

Un autre objet de l'invention est de fournir un procédé de synthèse permettant l'obtention d'un mélange de quatre *p*-(R)calix[9-12]arènes, en particulier de quatre *p*-(R-oxy)calix[9-12]arènes, ou de quelques *p*-(R)calix[9-12]arènes, en particulier de quelques *p*-(R-oxy)calix[9-12]arènes, isolés et purs, ou un mélange de deux ou trois, parmi les quatre, ainsi qu'une procédure de purification permettant de les obtenir purs, en particuliers exempts de *p*-(R)calixarènes géants, dont la taille est supérieure à 20, c'est-à-dire des *p*-(R)calixarènes comportant plus de 20 motifs de répétition.

La présente invention concerne ainsi un procédé de préparation :

- d'un grand *p*-(R)calixarène choisi parmi un *p*-(R)calix[9]arène, un *p*-(R)calix[10]arène, un *p*-(R)calix[11]arène, un *p*-(R)calix[12]arène, un *p*-(R)calix[13]arène, un *p*-(R)calix[14]arène, un *p*-(R)calix[15]arène, un *p*-(R)calix[16]arène, un *p*-(R)calix[17]arène, un *p*-(R)calix[18]arène, un *p*-(R)calix[19]arène, un *p*-(R)calix[20]arène, ou
- d'un mélange (a) d'au moins deux desdits grands *p*-(R)calixarènes dans lequel lesdits au moins deux grands *p*-(R)calixarènes sont présents dans ledit mélange à raison d'au moins 5% molaire chacun,

comprenant une étape de mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I) suivante :

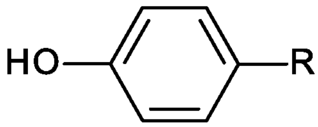

(I)

dans laquelle R est choisi parmi :

- un groupe benzyle éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyle en $C_1$-$C_{20}$ ramifié ou linéaire, à l'exclusion du groupe *t*-butyle,
- un groupe benzyloxy éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyloxy en $C_1$-$C_{20}$ ramifié ou linéaire,
- un groupe O-PEG 1 à 10 ramifié ou linéaire, dont le groupe terminal -OH est alkylé par un alkyle $C_1$-$C_{20}$ ramifié ou linéaire,
- un groupe benzylthioéther $-S-CH_2-Ph$ éventuellement substitué en ortho et/ou para et/ou méta par un ou plusieurs substituants choisis parmi un halogène, SR' et OR', $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkylthioéther en $C_1$-$C_{20}$ ramifié ou linéaire, de formule -S-($C_1$-$C_{20}$ alkyle),
- un groupe $-NR_aR_b$, $R_a$ et $R_b$ représentant indépendamment l'un de l'autre un alkyle $C_1$-$C_{20}$ ramifié ou linéaire,
- un groupe dibenzylamine de formule $-N(benzyle)_2$, dans lequel les deux groupes benzyle sont, indépendamment l'un de l'autre, éventuellement substitués en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,

et une source de formaldéhyde aqueux,

en l'absence de solvant organique, constituant ainsi un milieu réactionnel initial, ladite base étant à une concentration totale comprise d'environ 0,01 à 1 équivalent, notamment comprise d'environ 0,1 équivalent à environ 0,4 équivalent, en particulier égale à 0,15 ou 0,4 équivalent ou 1 équivalent, par rapport audit au moins un phénol substitué en position 4 de formule (I),

ledit milieu réactionnel initial étant chauffé à une température comprise de 100 à 165°C, pour obtenir un milieu réactionnel chauffé,

et comprenant, éventuellement, après ledit chauffage, une étape de traitement thermique supplémentaire dudit milieu

réactionnel chauffé, en présence de moyens de transmission de chaleur, notamment un four ou un liquide chauffé, pour obtenir un mélange (b), comprenant ledit grand *p*-(R)calixarène ou ledit mélange (a) d'au moins deux desdits grands *p*-(R)calixarènes, ledit mélange (b) comprenant de plus un mélange (c) de *p*-(R)calixarènes géants, dont la taille est supérieure à 20, ledit procédé comprenant en outre, après ledit chauffage et l'éventuel traitement thermique supplémentaire, une étape de purification dudit mélange (b), afin d'éliminer ledit mélange (c) de *p*-(R)calixarènes géants, et d'obtenir ledit grand *p*-(R)calixarène ou ledit mélange (a) d'au moins deux desdits grands *p*-(R)calixarènes, dépourvu dudit mélange (c) de *p*-(R)calixarènes géants.

De façon surprenante, les Inventeurs ont constaté la présence de grands calixarènes dans des fractions habituellement écartées lors de la purification de mélanges réactionnels ou de résidus comprenant des calixarènes géants.

De façon tout aussi surprenante, ces grands calixarènes ont une distribution de tailles originale par rapport à celles obtenues par les procédés d'obtention de grands calixarènes de l'art antérieur.

Par grand *p*-(R)calixarène, on entend un *p*-(R)calixarène choisi parmi un *p*-(R)calix[9]arène, un *p*-(R)calix[10] arène, un *p*-(R)calix[11]arène, un *p*-(R)calix[12]arène, un *p*-(R)calix[13]arène, un *p*-(R)calix[14]arène, un *p*-(R)calix[15] arène, un *p*-(R)calix[16] arène, *un p*-(R)calix[17]arène, un *p*-(R)calix[18]arène, un *p*-(R)calix[19]arène et un *p*-(R)calix[20] arène.

Par *p*-(R)calixarène géant, on entend un *p*-(R)calixarène dont la taille est supérieure à 20, c'est-à-dire un *p*-(R)calixarène comportant plus de 20 motifs de répétition. En d'autres termes, un *p*-(R)calixarène géant correspond à un *p*-(R)calix[Z]arène, avec $Z > 20$.

Par « dépourvu dudit mélange (c) de *p*-(R)calixarènes géants », on entend que ledit grand p-(R)calixarène ou ledit mélange (a) d'au moins deux desdits grands *p*-(R)calixarènes comprend moins de 10 % en masse de *p*-(R)calixarènes géants.

Le terme base désigne toute base soluble en milieu aqueux de type hydroxyde métallique, amine tertiaire, carbonate, sulfate, carboxylate, par exemple.

Il peut également désigner l'utilisation d'une base organique de type amine en combinaison avec un sel métallique (CsI par exemple).

L'expression « source de formaldéhyde aqueux » signifie que du formaldéhyde en solution aqueuse tel que de la formaline ou du formol peuvent être utilisés.

L'expression « en solution aqueuse » dans toute la description signifie que la base est dissoute dans un milieu majoritairement constitué d'eau.

Avantageusement, l'expression « en solution aqueuse » désigne exclusivement de l'eau.

L'expression « en l'absence de solvant organique » signifie que le milieu réactionnel (qui comprend de l'eau) est respectivement dépourvu de solvants qui sont des composés organiques liquides qui contiennent des atomes de carbone, la base en tant que telle, le formaldéhyde ou la source de formaldéhyde et le phénol n'étant pas non plus considérés comme solvant organique.

L'expression « milieu réactionnel » signifie le mélange des différents composants, comprenant la ou les base(s), le ou les phénol(s) substitué(s) en position 4 de formule (I), la source de formaldéhyde, le tout étant en solution aqueuse.

Lorsque ledit mélange vient d'être constitué, il est dénommé milieu réactionnel initial, c'est-à-dire un milieu réactionnel dans lequel la réaction de préparation des *p*-(R)calixarènes n'a pas encore été effectuée, en particulier avant tout chauffage dudit mélange des différents composés.

Le milieu réactionnel chauffé correspond au milieu réactionnel initial qui a été chauffé à une température comprise de 100°C à 165°C durant d'environ 20 minutes à 5 heures.

Lorsque la réaction de préparation des grands *p*-(R)calixarènes est effectuée, en particulier après chauffage dudit mélange des différents composés et traitement thermique supplémentaire, le milieu réactionnel est alors dénommé milieu réactionnel final.

L'expression « et éventuellement soumis ensuite à un traitement thermique supplémentaire en présence de moyens de transmission de chaleur» signifie qu'après ledit chauffage à une température comprise de 100°C à 165°C, au cours duquel l'eau est éventuellement éliminée, des moyens de transmission de chaleur sont appliqués au milieu réactionnel et ledit milieu réactionnel est chauffé à nouveau.

Les moyens de transmission de chaleur sont en particulier un four ou un liquide chauffé, en particulier un liquide chauffé à une température comprise de 100°C à 165°C, par exemple un liquide dont la température d'ébullition est comprise de 100°C à 165°C.

Par liquide, on entend un liquide, en particulier une huile de silicone ou un liquide organique.

Par très peu soluble, on entend une solubilité supérieure ou égale à 0,01 $g.L^{-1}$, mais inférieure à 1 $g.L^{-1}$.

Par totalement insoluble, on entend une solubilité inférieure à 0,01 $g.L^{-1}$.

Par « liquide dans lequel ledit milieu réactionnel ou ledit précurseur solide est très peu soluble», on entend ainsi un liquide pouvant éventuellement, selon sa nature, solubiliser partiellement certains des constituants dudit milieu réactionnel, les disperser (pour former une suspension colloïdale) ou faire passer tout ou partie d'entre eux à l'état de gel.

Ainsi, lorsque les moyens de transmission de chaleur sont par exemple un four ou un liquide dans lequel les

composés organiques du milieu réactionnel sont très peu solubles ou totalement insolubles, ledit traitement thermique supplémentaire se fait en phase solide, de préférence sans agitation.

Dans un autre mode de réalisation avantageux, le milieu réactionnel est dépourvu de solvant organique.

Le milieu réactionnel initial est ainsi chauffé en l'absence de solvant organique, la base en tant que telle, le formaldéhyde ou la source de formaldéhyde et le phénol n'étant pas non plus considérés comme solvants organiques.

L'expression « milieu réactionnel » signifie alors le mélange des différents composants, comprenant la ou les base(s), le ou les phénol(s) substitué(s) en position 4 de formule (I), la source de formaldéhyde, le tout étant en solution aqueuse.

Dans un autre mode de réalisation avantageux, le milieu réactionnel est dépourvu de solvant organique, l'eau est éliminée au cours dudit chauffage à une température comprise de 100 à 165°C, et ledit traitement thermique supplémentaire est réalisé en présence de moyens de transmission de chaleur.

Dans un autre mode de réalisation avantageux, le milieu réactionnel est dépourvu de solvant organique, l'eau n'est pas éliminée au cours dudit chauffage à une température comprise de 100 à 165°C, et ledit traitement thermique supplémentaire est réalisé en présence de moyens de transmission de chaleur.

Le procédé de l'invention permet en particulier d'obtenir une distribution de grands p-(R)calix[9-12]arène d'intérêt.

Dans un mode de réalisation avantageux, la présente invention concerne un procédé de préparation :

- d'un grand *p*-(R)calixarène choisi parmi un *p*-(R)calix[9]arène, un *p*-(R)calix[10] arène, un *p*-(R)calix[11]arène, un *p*-(R)calix[12]arène, ou
- d'un mélange (a) d'au moins deux desdits grands *p*-(R)calixarènes dans lequel lesdits au moins deux grands *p*-(R)calixarènes sont présents dans ledit mélange à raison d'au moins 5% molaire chacun.

Le procédé de l'invention permet en particulier d'obtenir des grands *p*-(alkyloxy)calix[9-20]arènes, *p*-(benzyloxy)calix[9-20]arènes ou *p*-(PEG-oxy)calix[9-20]arènes.

Dans un mode de réalisation avantageux, la présente invention concerne un procédé de préparation :

- d'un grand *p*-($R_1$-oxy)calixarène choisi parmi un *p*-($R_1$-oxy)calix[9]arène, un *p*-($R_1$-oxy)calix[10]arène, un *p*-($R_1$-oxy)calix[11]arène, un *p*-($R_1$-oxy)calix[12]arène, un *p*-($R_1$-oxy)calix[13]arène, un *p*-($R_1$-oxy)calix[14]arène, un *p*-($R_1$-oxy)calix[15]arène, un *p*-($R_1$-oxy)calix[16]arène, un *p*-($R_1$-oxy)calix[17]arène, un *p*-($R_1$-oxy)calix[18]arène, un *p*-($R_1$-oxy)calix[19]arène, un *p*-($R_1$-oxy)calix[20]arène, ou
- d'un mélange d'au moins deux desdits grands *p*-($R_1$-oxy)calixarènes dans lequel lesdits au moins deux grands *p*-($R_1$-oxy)calixarènes sont présents dans ledit mélange à raison d'au moins 5% molaire chacun,

comprenant une étape de mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (Ia) suivante :

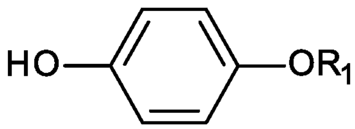

(Ia)

dans laquelle $R_1$ est choisi parmi :

- un groupe benzyle éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, NRaRb, PRaRb, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyle en $C_1$-$C_{20}$ ramifié ou linéaire,
- un PEG 1 à 10 ramifié ou linéaire, dont le groupe terminal -OH est alkylé par un alkyle $C_1$-$C_{20}$ ramifié ou linéaire,

et une source de formaldéhyde aqueux,
en l'absence de solvant organique, constituant ainsi un milieu réactionnel initial,
ladite base étant à une concentration totale comprise d'environ 0,01 à 1 équivalent, notamment comprise d'environ 0,1 équivalent à environ 0,4 équivalent, en particulier égale à 0,15 ou 0,4 équivalent ou 1 équivalent, par rapport audit au

moins un phénol substitué en position 4 de formule (I),
ledit milieu réactionnel initial étant chauffé à une température comprise de 100 à 165°C, pour obtenir un milieu réactionnel chauffé,
et comprenant, éventuellement, après ledit chauffage, une étape de traitement thermique supplémentaire dudit milieu réactionnel chauffé, en présence de moyens de transmission de chaleur, notamment un four ou un liquide chauffé, pour obtenir un mélange (b), comprenant ledit grand *p*-($R_1$-oxy)calixarène ou ledit mélange (a) d'au moins deux desdits grands *p*-($R_1$-oxy)calixarènes, ledit mélange (b) comprenant de plus un mélange (c) de *p*-($R_1$-oxy)calixarènes géants, dont la taille est supérieure à 20,
ledit procédé comprenant en outre, après ledit chauffage et l'éventuel traitement thermique supplémentaire, une étape de purification dudit mélange (b), afin d'éliminer ledit mélange (c) de *p*-($R_1$-oxy)calixarènes géants, et d'obtenir ledit grand *p*-($R_1$-oxy)calixarène ou ledit mélange (a) d'au moins deux desdits grands *p*-($R_1$-oxy)calixarènes, dépourvu dudit mélange (c) de *p*-($R_1$-oxy)calixarènes géants.

Le procédé de l'invention permet en particulier d'obtenir une distribution de grands *p*-($R_1$-oxy)calix[9-12]arène d'intérêt.

Dans un mode de réalisation avantageux, la présente invention concerne un procédé de préparation :

- d'un grand *p*-($R_1$-oxy)calixarène choisi parmi un *p*-($R_1$-oxy)calix[9]arène, un *p*-($R_1$-oxy)calix[10]arène, un *p*-($R_1$-oxy)calix[11]arène, un *p*-($R_1$-oxy)calix[12]arène, ou
- d'un mélange d'au moins deux desdits grands *p*-($R_1$-oxy)calixarènes dans lequel lesdits au moins deux grands *p*-($R_1$-oxy)calixarènes sont présents dans ledit mélange à raison d'au moins 5% molaire chacun.

Dans le procédé de l'invention, le milieu réactionnel initial comprend de l'eau, en particulier apportée par la source de formaldéhyde aqueux. En outre, il se forme de l'eau au cours de la réaction de formation des calixarènes.

L'eau présente dans le milieu réactionnel initial ainsi que celle produite lors de la réaction peuvent être éliminées dudit milieu réactionnel durant ledit chauffage.

Dans un mode de réalisation avantageux, la présente invention concerne un procédé dans lequel l'eau présente dans le milieu réactionnel initial ainsi que celle produite lors de la réaction sont éliminées dudit milieu réactionnel durant ledit chauffage et un précurseur solide sous forme de résine dure cassante, éventuellement isolé, est obtenu.

Ledit précurseur solide sous forme de résine dure cassante est en particulier obtenu par chauffage du milieu réactionnel à une température comprise de 100 à 165°C pendant un temps compris de 1 à 5 heures, tout en éliminant éventuellement l'eau présente dans le milieu réactionnel initial ainsi que celle formée au cours de la réaction.

Avantageusement, le chauffage est d'environ 110°C, 120°C, 130°C, 140°C, 150°C ou 160°C.

Dans ce cas, la proportion d'eau présente après obtention de la résine est de moins de 5% en poids.

Dans un mode de réalisation avantageux, ledit milieu réactionnel initial est chauffé durant un temps compris de 20 minutes à 2 heures, en particulier d'environ 30 minutes à environ 1 heure, sans éliminer l'eau présente dans le milieu réactionnel initial ainsi que celle formée, puis le chauffage dudit milieu réactionnel est poursuivi durant 1 à 3 heures tout en éliminant l'eau présente dans le milieu réactionnel ainsi que celle formée, pour obtenir un milieu réactionnel contenant un précurseur solide, sous forme de résine dure cassante.

Dans un mode de réalisation avantageux, ledit milieu réactionnel initial est chauffé durant 1 heure 20 minutes à 5 heures, tout en éliminant l'eau présente dans le milieu réactionnel ainsi que celle formée, pour obtenir un milieu réactionnel contenant un précurseur solide, sous forme de résine dure cassante.

Dans un mode de réalisation avantageux, la présente invention concerne un procédé dans lequel l'eau présente dans le milieu réactionnel initial ainsi que celle produite lors de la réaction sont éliminées dudit milieu réactionnel durant ledit chauffage et un précurseur solide sous forme de résine dure cassante, ledit précurseur solide sous forme de résine dure cassante étant isolé du susdit milieu réactionnel et dépourvu de traitement thermique supplémentaire.

Dans un mode de réalisation avantageux, la présente invention concerne un procédé dans lequel l'eau présente dans le milieu réactionnel initial ainsi que celle produite lors de la réaction sont éliminées dudit milieu réactionnel durant ledit chauffage et un précurseur solide sous forme de résine dure cassante est obtenu, ledit précurseur solide sous forme de résine dure cassante étant isolé du susdit milieu réactionnel et dépourvu de traitement thermique supplémentaire,
ledit procédé comprenant les étapes suivantes :

a. mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I), en particulier de formule (Ia),
avec du formaldéhyde aqueux, pour constituer un milieu réactionnel initial et chauffage dudit milieu réactionnel initial durant un temps compris de 20 minutes à 2 heures, en particulier d'environ 30 minutes à environ 1 heure, tout en éliminant éventuellement l'eau présente dans le milieu réactionnel initial ainsi que celle formée,

b. poursuite du chauffage durant 1 à 3 heures dudit milieu réactionnel tout en éliminant l'eau présente dans le milieu réactionnel ainsi que celle formée pour obtenir un milieu réactionnel contenant un précurseur solide, sous forme de résine dure cassante, ledit précurseur solide sous forme de résine dure cassante étant isolé du susdit milieu réactionnel,

pour obtenir un mélange (b) comprenant ledit grand *p*-(R)calixarène ou ledit mélange (a) d'au moins deux desdits grands *p*-(R)calixarènes, ledit mélange (b) comprenant de plus un mélange (c) de *p*-(R)calixarènes géants, dont la taille est supérieure à 20,

c. éventuellement, après neutralisation de la base, au moins un lavage dudit mélange (b) par un solvant polaire, en particulier le méthanol ou l'acétonitrile, pour obtenir ledit mélange (b), sous forme neutralisée,

d. purification dudit mélange (b) éventuellement neutralisé, afin d'éliminer ledit mélange (c) de *p*-(R)calixarènes géants, et d'obtenir ledit grand *p*-(R)calixarène ou ledit mélange (a) d'au moins deux desdits grands *p*-(R)calixarènes, dépourvu dudit mélange (c) de *p*-(R)calixarènes géants,

e. éventuellement, purification du mélange (a) par GPC, en séparant différentes fractions en fonction de leur temps d'élution, pour obtenir ledit grand p-(R)calixarène.

Dans l'étape c., le neutralisation peut par exemple être réalisée à l'aide d'un acide tel que HCl ou $H_2SO_4$, en particulier HCl dilué ou non.

L'étape e. peut être effectuée pour séparer ledit mélange (a) d'au moins deux desdits grands *p*-(R)calixarènes en fractions de grands *p*-(R)calixarènes de taille différente.

Dans un mode de réalisation avantageux, la présente invention concerne un procédé dans lequel l'eau présente dans le milieu réactionnel initial ainsi que celle produite lors de la réaction sont éliminées dudit milieu réactionnel durant ledit chauffage et un précurseur solide sous forme de résine dure cassante, éventuellement isolé, est obtenu,
dans lequel ledit précurseur solide sous forme de résine dure cassante, contenu dans ledit milieu réactionnel, est mis en présence de moyens de transmission de chaleur sous la forme d'un four, ou d'un liquide chauffé dans lequel ledit précurseur solide sous forme de résine dure cassante, est très peu soluble ou totalement insoluble, notamment dans le toluène, le xylène, un alcane linéaire ou ramifié de point d'ébullition supérieur à 50°C, notamment supérieur à 100°C, en particulier l'octane, ou une huile de silicone, et est soumis à un traitement thermique supplémentaire.

Le point d'ébullition dudit liquide est notamment supérieur ou égal à 110°C.

Le traitement thermique supplémentaire est notamment effectué au reflux dudit liquide, avec ou sans agitation, préférentiellement sans agitation du milieu réactionnel.

Le traitement thermique supplémentaire est notamment effectué durant un temps compris en fonction de R et de la base de 3 à 24h et a notamment pour effet d'une part de compléter la formation des macrocycles, notamment ceux compris dans le précurseur solide sous forme de résine.

Dans un procédé selon l'invention, ledit précurseur solide sous forme de résine dure cassante peut éventuellement être soumis à un traitement thermique supplémentaire, avant neutralisation éventuelle, et purification.

Dans un mode de réalisation avantageux, la présente invention concerne un procédé, comprenant les étapes suivantes :

a. mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I), en particulier de formule (Ia),
avec du formaldéhyde aqueux, pour constituer un milieu réactionnel initial et chauffage dudit milieu réactionnel initial durant un temps compris de 20 minutes à 2 heures, en particulier d'environ 30 minutes à environ 1 heure, tout en éliminant éventuellement l'eau présente dans le milieu réactionnel initial ainsi que celle formée,

b. poursuite du chauffage durant 1 à 3 heures dudit milieu réactionnel tout en éliminant l'eau présente dans le milieu réactionnel ainsi que celle formée pour obtenir un milieu réactionnel contenant un précurseur solide, sous forme de résine dure cassante,

c. éventuellement :

◦ chauffage dudit milieu réactionnel ou dudit précurseur solide à l'aide de moyens de transmission de chaleur sous la forme d'un four, durant 3 à 24 heures,

◦ ou addition audit milieu réactionnel ou audit précurseur solide sous forme de résine dure cassante d'un liquide dans lequel ledit précurseur solide sous forme de résine dure cassante est très peu soluble ou totalement insoluble, notamment dans le toluène, le xylène, un alcane linéaire ou ramifié de point d'ébullition supérieur à 50°C, notamment supérieur à 100°C, en particulier l'octane ou une huile de silicone, pour obtenir un milieu réactionnel contenant un solide sous forme de résine dure cassante, et chauffage dudit milieu réactionnel contenant un solide sous forme de résine dure cassante, durant 3 à 24 heures, avec ou sans agitation,

pour obtenir un mélange (b) comprenant ledit grand *p*-(R)calixarène ou ledit mélange (a) d'au moins deux desdits grands *p*-(R)calixarènes, ledit mélange (b) comprenant de plus un mélange (c) de *p*-(R)calixarènes géants, dont la taille est supérieure à 20,

d. éventuellement, après neutralisation de la base, au moins un lavage dudit mélange (b) par un solvant polaire, en particulier le méthanol ou l'acétonitrile, pour obtenir ledit mélange (b), sous forme neutralisée,

e. purification dudit mélange (b) éventuellement neutralisé, afin d'éliminer ledit mélange (c) de *p*-(R)calixarènes géants, et d'obtenir ledit grand *p*-(R)calixarène ou ledit mélange (a) d'au moins deux desdits grands *p*-(R)calixarènes, dépourvu dudit mélange (c) de *p*-(R)calixarènes géants,

f. éventuellement, purification du mélange (a) par GPC, en séparant différentes fractions en fonction de leur temps d'élution, pour obtenir ledit grand p-(R)calixarène.

Dans un procédé selon l'invention, ledit précurseur solide sous forme de résine dure cassante est soumis à un traitement thermique supplémentaire, avant neutralisation éventuelle, et purification.

Dans un mode de réalisation avantageux, la présente invention concerne un procédé, comprenant les étapes suivantes :

a. mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I), en particulier de formule (Ia),

avec du formaldéhyde aqueux, pour constituer un milieu réactionnel initial et chauffage dudit milieu réactionnel initial durant un temps compris de 20 minutes à 2 heures, en particulier d'environ 30 minutes à environ 1 heure, tout en éliminant éventuellement l'eau présente dans le milieu réactionnel initial ainsi que celle formée,

b. poursuite du chauffage durant 1 à 3 heures dudit milieu réactionnel tout en éliminant l'eau présente dans le milieu réactionnel ainsi que celle formée pour obtenir un milieu réactionnel contenant un précurseur solide, sous forme de résine dure cassante,

c. chauffage dudit milieu réactionnel ou dudit précurseur solide à l'aide de moyens de transmission de chaleur sous la forme d'un four, durant 3 à 24 heures,

ou addition audit milieu réactionnel ou audit précurseur solide sous forme de résine dure cassante d'un liquide dans lequel ledit précurseur solide sous forme de résine dure cassante est très peu soluble ou totalement insoluble, notamment dans le toluène, le xylène, un alcane linéaire ou ramifié de point d'ébullition supérieur à 50°C, notamment supérieur à 100°C, en particulier l'octane ou une huile de silicone, pour obtenir un milieu réactionnel contenant un solide sous forme de résine dure cassante, et chauffage dudit milieu réactionnel contenant un solide sous forme de résine dure cassante, durant 3 à 24 heures, avec ou sans agitation, pour obtenir un mélange (b) comprenant ledit grand *p*-(R)calixarène ou ledit mélange (a) d'au moins deux desdits grands *p*-(R)calixarènes, ledit mélange (b) comprenant de plus un mélange (c) de *p*-(R)calixarènes géants, dont la taille est supérieure à 20,

d. éventuellement, après neutralisation de la base, au moins un lavage dudit mélange (b) par un solvant polaire, en particulier le méthanol ou l'acétonitrile, pour obtenir ledit mélange (b), sous forme neutralisée,

e. purification dudit mélange (b) éventuellement neutralisé, afin d'éliminer ledit mélange (c) de *p*-(R)calixarènes géants, et d'obtenir ledit grand *p*-(R)calixarène ou ledit mélange (a) d'au moins deux desdits grands *p*-(R)calixarènes, dépourvu dudit mélange (c) de *p*-(R)calixarènes géants,

f. éventuellement, purification du mélange (a) par GPC, en séparant différentes fractions en fonction de leur temps d'élution, pour obtenir ledit grand p-(R)calixarène.

Avantageusement, dans les étapes a. et b., le chauffage est d'environ 110°C, 120°C, 130°C, 140°C, 150°C ou 160°C.

En particulier, le traitement thermique supplémentaire est effectué au reflux dudit liquide, avec ou sans agitation, préférentiellement sans agitation du milieu réactionnel.

Le traitement thermique supplémentaire permet de compléter la formation des macrocycles.

Avantageusement, le traitement thermique supplémentaire est compris d'environ 3 à 24h.

L'étape f. peut être effectuée pour séparer ledit mélange (a) d'au moins deux desdits grands *p*-(R)calixarènes en fractions de grands *p*-(R)calixarènes de taille différente.

Dans un mode de réalisation avantageux, la base utilisée dans l'étape a. est $Ba(OH)_2$, LiOH, KOH, NaOH, CsOH ou RbOH et R représente le benzyloxy.

Dans un procédé selon l'invention, ledit précurseur solide sous forme de résine dure cassante est soumis à un traitement thermique supplémentaire dans un four, avant neutralisation éventuelle, et purification.

Dans un mode de réalisation avantageux, la présente invention concerne un procédé, comprenant les étapes suivantes :

a. mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I), en particulier de formule (Ia),
avec du formaldéhyde aqueux, pour constituer un milieu réactionnel initial et chauffage dudit milieu réactionnel initial durant un temps compris de 20 minutes à 2 heures, en particulier d'environ 30 minutes à environ 1 heure, tout en éliminant éventuellement l'eau présente dans le milieu réactionnel initial ainsi que celle formée,
b. poursuite du chauffage durant 1 à 3 heures dudit milieu réactionnel tout en éliminant l'eau présente dans le milieu réactionnel ainsi que celle formée pour obtenir un milieu réactionnel contenant un précurseur solide, sous forme de résine dure cassante,
c. chauffage dudit milieu réactionnel ou dudit précurseur solide à l'aide de moyens de transmission de chaleur sous la forme d'un four, durant 3 à 24 heures, pour obtenir un mélange (b) comprenant ledit grand *p*-(R)calixarène ou ledit mélange (a) d'au moins deux desdits grands *p*-(R)calixarènes, ledit mélange (b) comprenant de plus un mélange (c) de *p*-(R)calixarènes géants, dont la taille est supérieure à 20,
d. éventuellement, après neutralisation de la base, au moins un lavage dudit mélange (b) par un solvant polaire, en particulier le méthanol ou l'acétonitrile, pour obtenir ledit mélange (b), sous forme neutralisée,
e. purification dudit mélange (b) éventuellement neutralisé, afin d'éliminer ledit mélange (c) de *p*-(R)calixarènes géants, et d'obtenir ledit grand *p*-(R)calixarène ou ledit mélange (a) d'au moins deux desdits grands *p*-(R)calixarènes, dépourvu dudit mélange (c) de *p*-(R)calixarènes géants,
f. éventuellement, purification du mélange (a) par GPC, en séparant différentes fractions en fonction de leur temps d'élution, pour obtenir ledit grand p-(R)calixarène.

Dans un procédé selon l'invention, ledit précurseur solide sous forme de résine dure cassante est soumis à un traitement thermique supplémentaire dans un liquide chauffé, avant neutralisation éventuelle, et purification.
Dans un mode de réalisation avantageux, la présente invention concerne un procédé, comprenant les étapes suivantes :

a. mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I), en particulier de formule (Ia),
avec du formaldéhyde aqueux, pour constituer un milieu réactionnel initial et chauffage dudit milieu réactionnel initial durant un temps compris de 20 minutes à 2 heures, en particulier d'environ 30 minutes à environ 1 heure, tout en éliminant éventuellement l'eau présente dans le milieu réactionnel initial ainsi que celle formée,
b. poursuite du chauffage durant 1 à 3 heures dudit milieu réactionnel tout en éliminant l'eau présente dans le milieu réactionnel ainsi que celle formée pour obtenir un milieu réactionnel contenant un précurseur solide, sous forme de résine dure cassante,
c. addition audit milieu réactionnel ou audit précurseur solide sous forme de résine dure cassante d'un liquide dans lequel ledit précurseur solide sous forme de résine dure cassante est très peu soluble ou totalement insoluble, notamment dans le toluène, le xylène, un alcane linéaire ou ramifié de point d'ébullition supérieur à 50°C, notamment supérieur à 100°C, en particulier l'octane ou une huile de silicone, pour obtenir un milieu réactionnel contenant un solide sous forme de résine dure cassante, et chauffage dudit milieu réactionnel contenant un solide sous forme de résine dure cassante, durant 3 à 24 heures, avec ou sans agitation, pour obtenir un mélange (b) comprenant ledit grand *p*-(R)calixarène ou ledit mélange (a) d'au moins deux desdits grands *p*-(R)calixarènes, ledit mélange (b) comprenant de plus un mélange (c) de *p*-(R)calixarènes géants, dont la taille est supérieure à 20,
d. éventuellement, après neutralisation de la base, au moins un lavage dudit mélange (b) par un solvant polaire, en particulier le méthanol ou l'acétonitrile, pour obtenir ledit mélange (b), sous forme neutralisée,
e. purification dudit mélange (b) éventuellement neutralisé, afin d'éliminer ledit mélange (c) de *p*-(R)calixarènes géants, et d'obtenir ledit grand *p*-(R)calixarène ou ledit mélange (a) d'au moins deux desdits grands *p*-(R)calixarènes, dépourvu dudit mélange (c) de *p*-(R)calixarènes géants,
f. éventuellement, purification du mélange (a) par GPC, en séparant différentes fractions en fonction de leur temps d'élution, pour obtenir ledit grand p-(R)calixarène.

Dans le procédé de l'invention, le milieu réactionnel initial comprend de l'eau, en particulier apportée par la source de formaldéhyde aqueux.En outre, il se forme de l'eau au cours de la réaction de formation des calixarènes.
L'eau présente dans le milieu réactionnel initial ainsi que celle produite lors de la réaction peuvent être conservées dans ledit milieu réactionnel durant ledit chauffage.
Dans un mode de réalisation avantageux, la présente invention concerne un procédé, dans lequel l'eau présente dans le milieu réactionnel initial ainsi que celle produite lors de la réaction sont conservées dans le milieu réactionnel durant ledit chauffage et un précurseur solide sous forme de précipité, éventuellement isolé, est obtenu.

Dans ce mode de réalisation, l'eau présente dans le milieu réactionnel initial ainsi que celle produite lors de la réaction ne sont pas éliminées mais conservée dans le milieu réactionnel.
Ceci a pour conséquence que le milieu réactionnel ne se solidifie pas complètement et qu'un précipité est obtenu. Le précipité, de la même manière que la résine dure cassante, peut être isolé par simple filtration.
Dans un mode de réalisation avantageux, ledit milieu réactionnel initial est chauffé durant un temps compris de 20 minutes à 5 heures, en particulier d'environ 30 minutes à environ 2 heures.
Dans un mode de réalisation avantageux, la présente invention concerne un procédé, dans lequel l'eau présente dans le milieu réactionnel initial ainsi que celle produite lors de la réaction sont conservées dans le milieu réactionnel durant ledit chauffage et un précurseur solide sous forme de précipité est obtenu, ledit précurseur solide sous forme de précipité étant isolé du susdit milieu réactionnel et dépourvu de traitement thermique supplémentaire.
Dans un mode de réalisation avantageux, la présente invention concerne un procédé comprenant les étapes suivantes :

a. mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I), en particulier de formule (Ia),
avec du formaldéhyde aqueux, pour constituer un milieu réactionnel initial et chauffage dudit milieu réactionnel initial durant un temps compris de 20 minutes à 5 heures, en particulier d'environ 30 minutes à environ 2 heures, sans élimination de l'eau présente dans le milieu réactionnel initial ainsi que de celle formée, pour obtenir un milieu réactionnel contenant un précurseur solide, sous forme de précipité,
pour obtenir un mélange (b) comprenant ledit grand *p*-(R)calixarène ou ledit mélange (a) d'au moins deux desdits grands *p*-(R)calixarènes, ledit mélange (b) comprenant de plus un mélange (c) de *p*-(R)calixarènes géants, dont la taille est supérieure à 20,
b. éventuellement, après neutralisation de la base, au moins un lavage dudit mélange (b) par un solvant polaire, en particulier le méthanol ou l'acétonitrile, pour obtenir ledit mélange (b), sous forme neutralisée,
c. purification dudit mélange (b) éventuellement neutralisé, afin d'éliminer ledit mélange (c) de *p*-(R)calixarènes géants, et d'obtenir ledit grand *p*-(R)calixarène ou ledit mélange (a) d'au moins deux desdits grands *p*-(R)calixarènes, dépourvu dudit mélange (c) de *p*-(R)calixarènes géants,
d. éventuellement, purification du mélange (a) par GPC, en séparant différentes fractions en fonction de leur temps d'élution, pour obtenir ledit grand p-(R)calixarène.

Dans l'étape c., le neutralisation peut par exemple être réalisée à l'aide d'un acide tel que HCl ou $H_2SO_4$, en particulier HCl dilué ou non.
L'étape d. peut être effectuée pour séparer ledit mélange (a) d'au moins deux desdits grands *p*-(R)calixarènes en fractions de grands *p*-(R)calixarènes de taille différente.
Dans un mode de réalisation avantageux, la présente invention concerne un procédé, dans lequel l'eau présente dans le milieu réactionnel initial ainsi que celle produite lors de la réaction sont conservées dans le milieu réactionnel durant ledit chauffage et un précurseur solide sous forme de précipité, éventuellement isolé, est obtenu,
dans lequel ledit précurseur solide sous forme de résine dure cassante, contenu dans ledit milieu réactionnel, est mis en présence de moyens de transmission de chaleur sous la forme d'un four, ou d'un liquide chauffé dans lequel ledit précurseur solide sous forme de résine dure cassante, est très peu soluble ou totalement insoluble, notamment dans le toluène, le xylène, un alcane linéaire ou ramifié de point d'ébullition supérieur à 50°C, notamment supérieur à 100°C, en particulier l'octane, ou une huile de silicone, et est soumis à un traitement thermique supplémentaire.
Le point d'ébullition dudit liquide est notamment supérieur ou égal à 110°C.
Le traitement thermique supplémentaire est notamment effectué au reflux dudit liquide, avec ou sans agitation, préférentiellement sans agitation du milieu réactionnel.
Le traitement thermique supplémentaire est notamment effectué durant un temps compris en fonction de R et de la base de 3 à 24h et a notamment pour effet de compléter la formation des macrocycles, notamment ceux compris dans le précurseur solide sous forme de résine.
Dans un procédé selon l'invention, ledit précurseur solide sous forme de précipité peut éventuellement être soumis à un traitement thermique supplémentaire, avant neutralisation éventuelle, et purification.
Dans un mode de réalisation avantageux, la présente invention concerne un procédé, comprenant les étapes suivantes :

a. mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I), en particulier de formule (Ia),
avec du formaldéhyde aqueux, pour constituer un milieu réactionnel initial et chauffage dudit milieu réactionnel initial

durant un temps compris de 20 minutes à 5 heures, en particulier d'environ 30 minutes à environ 2 heures, sans élimination

de l'eau présente dans le milieu réactionnel initial ainsi que de celle formée, pour obtenir un milieu réactionnel contenant un précurseur solide, sous forme de précipité,

b. éventuellement, addition audit milieu réactionnel ou audit précurseur solide sous forme de précipité d'un liquide dans lequel ledit précurseur solide sous forme de précipité est très peu soluble ou totalement insoluble, notamment dans le toluène le xylène, un alcane linéaire ou ramifié de point d'ébullition supérieur à 50°C, notamment supérieur à 100°C, en particulier l'octane ou une huile de silicone, pour obtenir un milieu réactionnel contenant un solide sous forme de précipité, et chauffage dudit milieu réactionnel contenant un solide sous forme de précipité, durant 3 à 24 heures, avec ou sans agitation,

pour obtenir un mélange (b) comprenant ledit grand *p*-(R)calixarène ou ledit mélange (a) d'au moins deux desdits grands *p*-(R)calixarènes, ledit mélange (b) comprenant de plus un mélange (c) de *p*-(R)calixarènes géants, dont la taille est supérieure à 20,

c. éventuellement, après neutralisation de la base, au moins un lavage dudit mélange (b) par un solvant polaire, en particulier le méthanol ou l'acétonitrile, pour obtenir ledit mélange (b), sous forme neutralisée,

d. purification dudit mélange (b) éventuellement neutralisé, afin d'éliminer ledit mélange (c) de *p*-(R)calixarènes géants, et d'obtenir ledit grand *p*-(R)calixarène ou ledit mélange (a) d'au moins deux desdits grands *p*-(R)calixarènes, dépourvu dudit mélange (c) de *p*-(R)calixarènes géants,

e. éventuellement, purification du mélange (a) par GPC, en séparant différentes fractions en fonction de leur temps d'élution, pour obtenir ledit grand p-(R)calixarène.

Dans un procédé selon l'invention, ledit précurseur solide sous forme de précipité est soumis à un traitement thermique supplémentaire, avant neutralisation éventuelle, et purification.

Dans un mode de réalisation avantageux, la présente invention concerne un procédé comprenant notamment les étapes suivantes :

a. mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I), en particulier de formule (Ia),

avec du formaldéhyde aqueux, pour constituer un milieu réactionnel initial et chauffage dudit milieu réactionnel initial durant un temps compris de 20 minutes à 5 heures, en particulier d'environ 30 minutes à environ 2 heures, sans élimination de l'eau présente dans le milieu réactionnel initial ainsi que de celle formée, pour obtenir un milieu réactionnel contenant un précurseur solide, sous forme de précipité,

b. addition audit milieu réactionnel ou audit précurseur solide sous forme de précipité d'un liquide dans lequel ledit précurseur solide sous forme de précipité est très peu soluble ou totalement insoluble, notamment dans le toluène, le xylène, un alcane linéaire ou ramifié de point d'ébullition supérieur à 50°C, notamment supérieur à 100°C, en particulier l'octane ou une huile de silicone, pour obtenir un milieu réactionnel contenant un solide sous forme de précipité, et chauffage dudit milieu réactionnel contenant un solide sous forme de précipité, durant 3 à 24 heures, avec ou sans agitation,

pour obtenir un mélange (b) comprenant ledit grand *p*-(R)calixarène ou ledit mélange (a) d'au moins deux desdits grands *p*-(R)calixarènes, ledit mélange (b) comprenant de plus un mélange (c) de *p*-(R)calixarènes géants, dont la taille est supérieure à 20,

c. éventuellement, après neutralisation de la base, au moins un lavage dudit mélange (b) par un solvant polaire, en particulier le méthanol ou l'acétonitrile, pour obtenir ledit mélange (b), sous forme neutralisée,

d. purification dudit mélange (b) éventuellement neutralisé, afin d'éliminer ledit mélange (c) de *p*-(R)calixarènes géants, et d'obtenir ledit grand *p*-(R)calixarène ou ledit mélange (a) d'au moins deux desdits grands *p*-(R)calixarènes, dépourvu dudit mélange (c) de *p*-(R)calixarènes géants,

e. éventuellement, purification du mélange (a) par GPC, en séparant différentes fractions en fonction de leur temps d'élution, pour obtenir ledit grand p-(R)calixarène.

Avantageusement, dans les étapes a. et b., le chauffage est d'environ 110°C, 120°C, 130°C, 140°C, 150°C ou 160°C.

En particulier, le traitement thermique supplémentaire est effectué au reflux dudit liquide, avec ou sans agitation, préférentiellement sans agitation du milieu réactionnel.

Le traitement thermique supplémentaire permet de compléter la formation des macrocycles.

Avantageusement, le traitement thermique supplémentaire est compris d'environ 3 à 24h.

L'étape e. peut être effectuée pour séparer ledit mélange (a) d'au moins deux desdits grands *p*-(R)calixarènes en fractions de grands *p*-(R)calixarènes de taille différente.

Dans un mode de réalisation avantageux, la base utilisée dans l'étape a. est $Ba(OH)_2$, LiOH, KOH, NaOH, CsOH ou RbOH et R représente le benzyloxy.

Dans un procédé selon l'invention, le milieu réactionnel comprend de l'eau et un liquide, ledit liquide étant différent de l'eau.

Dans un mode de réalisation avantageux, la présente description divulgue un procédé, dans lequel ledit milieu réactionnel initial comprend en outre un liquide, en particulier un solvant organique, plus particulièrement le toluène, le xylène, un alcane linéaire ou ramifié de point d'ébullition supérieur à 50°C, notamment supérieur à 100°C, encore plus particulièrement l'octane, ou une huile de silicone.

Avantageusement, le chauffage est d'environ 110°C, 120°C, 130°C, 140°C, 150°C ou 160°C.

Dans un mode de réalisation avantageux, ledit milieu réactionnel initial est chauffé durant un temps compris de 3 heures à 24 heures, en éliminant l'eau présente dans le milieu réactionnel initial ainsi que celle formée.

Dans un mode de réalisation avantageux, ledit milieu réactionnel initial est chauffé durant un temps compris de 3 heures à 24 heures, sans éliminer l'eau présente dans le milieu réactionnel initial ainsi que celle formée.

Dans un mode de réalisation avantageux, la présente description divulgue un procédé comprenant notamment les étapes suivantes :

a. mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I), en particulier de formule (Ia),
avec du formaldéhyde aqueux et un liquide, en particulier un solvant organique, plus particulièrement le toluène, le xylène, un alcane linéaire ou ramifié de point d'ébullition supérieur à 50°C, notamment supérieur à 100°C, encore plus particulièrement l'octane, ou une huile de silicone,
pour constituer un milieu réactionnel initial et chauffage dudit milieu réactionnel initial durant un temps compris de 1 heure à 5 heures, en particulier d'environ 1 heure à environ 2 heures, sans élimination de l'eau présente dans le milieu réactionnel initial ainsi que de celle formée, pour obtenir un milieu réactionnel contenant un précurseur solide, sous forme de précipité,
pour obtenir un mélange (b) comprenant ledit grand *p*-(R)calixarène ou ledit mélange (a) d'au moins deux desdits grands *p*-(R)calixarènes, ledit mélange (b) comprenant de plus un mélange (c) de *p*-(R)calixarènes géants, dont la taille est supérieure à 20,
b. éventuellement, après neutralisation de la base, au moins un lavage dudit mélange (b) par un solvant polaire, en particulier le méthanol ou l'acétonitrile, pour obtenir ledit mélange (b), sous forme neutralisée,
c. purification dudit mélange (b) éventuellement neutralisé, afin d'éliminer ledit mélange (c) de *p*-(R)calixarènes géants, et d'obtenir ledit grand *p*-(R)calixarène ou ledit mélange (a) d'au moins deux desdits grands *p*-(R)calixarènes, dépourvu dudit mélange (c) de *p*-(R)calixarènes géants,
d. éventuellement, purification du mélange (a) par GPC, en séparant différentes fractions en fonction de leur temps d'élution, pour obtenir ledit grand p-(R)calixarène.

Dans l'étape b., le neutralisation peut par exemple être réalisée à l'aide d'un acide tel que HCl ou $H_2SO_4$, en particulier HCl dilué ou non.

L'étape d. peut être effectuée pour séparer ledit mélange (a) d'au moins deux desdits grands *p*-(R)calixarènes en fractions de grands *p*-(R)calixarènes de taille différente.

Dans un mode de réalisation avantageux, la présente invention concerne un procédé dans lequel ladite purification dudit mélange (b) comprend une étape de recristallisation dudit mélange (b) pour obtenir à l'issue de l'étape de recristallisation un précipité et un filtrat, ledit filtrat comprenant ledit grand *p*-(R)calixarène ou ledit mélange (a) d'au moins deux desdits grands p-(R)calixarènes, dépourvu dudit mélange (c) de *p*-(R)calixarènes géants, et le précipité comprenant ledit mélange (c) de *p*-(R)calixarènes géants, dont la taille est supérieure à 20.

Dans un mode de réalisation avantageux, la présente invention concerne un procédé dans lequel ladite purification dudit mélange (b) comprend une étape de recristallisation à deux solvants dudit mélange (b) pour obtenir, après addition du deuxième solvant, un précipité et un filtrat, ledit filtrat comprenant ledit grand *p*-(R)calixarène ou ledit mélange (a) d'au moins deux desdits grands *p*-(R)calixarènes, et le mélange (c) de *p*-(R)calixarènes géants, et le précipité comprenant principalement du calix[8]arène.

Dans l'étape de recristallisation à deux solvants, bien connue de l'homme du métier, le premier solvant est tel que le mélange (c) est soluble à chaud dans ledit premier solvant. Le deuxième solvant est tel que le mélange (c) est très peu soluble ou insoluble dans ledit deuxième solvant.

Dans un procédé selon l'invention, ledit grand *p*-(R)calixarène ou ledit mélange (a) est obtenu par dissolution du mélange (b) dans le minimum d'un premier solvant, à chaud, ajout d'un deuxième solvant jusqu'à apparition d'un précipité, filtration immédiate, et après refroidissement du filtrat précédemment obtenu, deuxième filtration pour obtenir un filtrat comprenant ledit grand *p*-(R)calixarène ou ledit mélange (a), dépourvu dudit mélange (c) de *p*-(R)calixarènes

géants.

Dans un mode de réalisation avantageux, la présente invention concerne un procédé, dans lequel ladite purification dudit mélange (b) comprend les étapes suivantes :

- dissolution dudit mélange (b) dans le minimum d'un premier solvant, à chaud, pour obtenir une solution, à chaud, dudit mélange (b),
- ajout, à la solution, à chaud, dudit mélange (b), d'un deuxième solvant jusqu'à apparition d'un précipité, et filtration immédiate, pour obtenir un filtrat contenant les grands calixarènes et les calixarènes géants,
- après refroidissement du filtrat obtenu précédemment, filtration dudit précipité pour obtenir :
    - un filtrat comprenant ledit grand *p*-(R)calixarène ou ledit mélange (a) d'au moins deux desdits grands *p*-(R)calixarènes, dépourvu dudit mélange (c) de *p*-(R)calixarènes géants, et
    - le précipité isolé, comprenant ledit mélange (c) de *p*-(R)calixarènes géants, dont la taille est supérieure à 20.

Dans un mode de réalisation avantageux, la présente invention concerne un procédé, dans lequel ladite purification dudit mélange (b) comprend les étapes suivantes :

- dissolution dudit mélange (b) dans le minimum d'un premier solvant, à chaud, pour obtenir une solution, à chaud, dudit mélange (b),
- ajout, à la solution, à chaud, dudit mélange (b), d'un deuxième solvant jusqu'à apparition d'un premier précipité,
- filtration à chaud dudit premier précipité pour obtenir un filtrat,
- refroidissement du filtrat pour obtenir la formation d'un deuxième précipité,
- filtration dudit deuxième précipité pour obtenir :
    - un filtrat comprenant ledit grand *p*-(R)calixarène ou ledit mélange (a) d'au moins deux desdits grands *p*-(R)calixarènes, dépourvu dudit mélange (c) de *p*-(R)calixarènes géants, et
    - le précipité isolé, comprenant ledit mélange (c) de *p*-(R)calixarènes géants, dont la taille est supérieure à 20.

Dans un procédé selon l'invention, ledit grand *p*-(R)calixarène ou ledit mélange (a) est obtenu par dissolution du mélange (b) dans le minimum d'un premier solvant, à chaud, ajout d'un deuxième solvant jusqu'à apparition d'un précipité, filtration immédiate à chaud, refroidissement du filtrat puis apparition d'un précipité, nouvelle filtration et concentration du filtrat pour obtenir ledit grand *p*-(R)calixarène ou ledit mélange (a), dépourvu dudit mélange (c) de *p*-(R)calixarènes géants.

Dans un mode de réalisation avantageux, la présente invention concerne un procédé, dans lequel ladite purification dudit mélange (b) comprend les étapes suivantes :

- dissolution dudit mélange (b) dans le minimum d'un premier solvant, à chaud, pour obtenir une solution, à chaud, dudit mélange (b),
- ajout, à la solution, à chaud, dudit mélange (b), d'un deuxième solvant jusqu'à apparition d'un premier précipité,
- filtration à chaud dudit premier précipité pour obtenir un filtrat,
- refroidissement du filtrat pour obtenir la formation d'un deuxième précipité,
- filtration dudit deuxième précipité pour obtenir :
    - un filtrat comprenant ledit grand *p*-(R)calixarène ou ledit mélange (a) d'au moins deux desdits grands *p*-(R)calixarènes, dépourvu dudit mélange (c) de *p*-(R)calixarènes géants, et
    - le précipité isolé, comprenant ledit mélange (c) de *p*-(R)calixarènes géants, dont la taille est supérieure à 20,
- concentration dudit filtrat pour obtenir ledit grand *p*-(R)calixarène ou ledit mélange (a) d'au moins deux desdits grands *p*-(R)calixarènes, dépourvu dudit mélange (c) de *p*-(R)calixarènes géants.

Dans un procédé selon l'invention, ledit grand *p*-(R)calixarène ou ledit mélange (a) est obtenu par dissolution du mélange (b) dans le minimum de DMSO, à chaud, ajout d'un deuxième solvant moyennement polaire, en particulier l'acétone, jusqu'à apparition d'un précipité, filtration immédiate à chaud, refroidissement du filtrat puis apparition d'un précipité, nouvelle filtration et concentration du filtrat pour obtenir ledit grand *p*-(R)calixarène ou ledit mélange (a), dépourvu dudit mélange (c) de *p*-(R)calixarènes géants.

Dans un mode de réalisation avantageux, la présente invention concerne un procédé, dans lequel ladite purification dudit mélange (b) comprend les étapes suivantes :

- dissolution dudit mélange (b) dans le minimum de DMSO, à une température comprise d'environ 80°C à environ 160°C, en particulier d'environ 100°C à environ 140°C, plus particulièrement à une température d'environ 120°C, pour obtenir une solution, à chaud, dudit mélange (b),
- ajout, à la solution, à chaud, dudit mélange (b), d'un deuxième solvant moyennement polaire, en particulier l'acétone, jusqu'à apparition d'un premier précipité,
- filtration à chaud dudit premier précipité pour obtenir un filtrat,
- refroidissement du filtrat à environ 0°C pour obtenir la formation d'un deuxième précipité,
- filtration dudit deuxième précipité pour obtenir :

    ◦ un filtrat comprenant ledit grand *p*-(R)calixarène ou ledit mélange (a) d'au moins deux desdits grands *p*-(R)calixarènes, dépourvu dudit mélange (c) de *p*-(R)calixarènes géants, et
    ◦ le précipité isolé, comprenant ledit mélange (c) de *p*-(R)calixarènes géants, dont la taille est supérieure à 20,

- concentration dudit filtrat pour obtenir ledit grand *p*-(R)calixarène ou ledit mélange (a) d'au moins deux desdits grands *p*-(R)calixarènes, dépourvu dudit mélange (c) de *p*-(R)calixarènes géants.

Par solvant moyennement polaire, on entend un solvant comportant une constante diélectrique comprise entre 5 et 25.

Dans un mode de réalisation avantageux, la présente invention concerne un procédé, dans lequel ladite purification dudit mélange (b) comprend les étapes suivantes :

- dissolution dudit mélange (b) dans le minimum de DMSO, à une température comprise d'environ 80°C à environ 160°C, en particulier d'environ 100°C à environ 140°C, plus particulièrement à une température d'environ 120°C, pour obtenir une solution, à chaud, dudit mélange (b),
- ajout, à la solution, à chaud, dudit mélange (b), d'un deuxième solvant moyennement polaire, en particulier l'acétone, jusqu'à apparition d'un premier précipité,
- filtration à chaud dudit premier précipité pour obtenir un filtrat,
- refroidissement du filtrat à environ 0°C pour obtenir la formation d'un deuxième précipité,
- filtration dudit deuxième précipité pour obtenir :

    ◦ un filtrat comprenant ledit grand *p*-(R)calixarène ou ledit mélange (a) d'au moins deux desdits grands *p*-(R)calixarènes, dépourvu dudit mélange (c) de *p*-(R)calixarènes géants, et
    ◦ le précipité isolé, comprenant ledit mélange (c) de *p*-(R)calixarènes géants, dont la taille est supérieure à 20,

- concentration dudit filtrat pour obtenir un résidu dépourvu d'acétone,
- ajout audit résidu d'un solvant polaire, en particulier l'éthanol, notamment à température ambiante, pour obtenir une solution dudit résidu dans ledit solvant polaire,
- refroidissement de ladite solution dudit résidu, en particulier à environ 0°C, pour obtenir une suspension, ladite suspension étant filtrée pour obtenir un filtrat, ledit filtrat étant concentré pour obtenir ledit grand *p*-(R)calixarène ou ledit mélange (a) d'au moins deux desdits grands *p*-(R)calixarènes, dépourvu dudit mélange (c) de *p*-(R)calixarènes géants.

## EXEMPLES

### Exemple 1 : Synthèse de grands calixarènes, avec traitement thermique supplémentaire dans une huile de silicone

Dans un ballon tricol de 2 1 équipé d'un agitateur mécanique, on introduit 104g de *p*-(benzyloxy)phénol, 133ml de formaldehyde en solution aqueuse à 37%, et 36ml de CsOH en solution aqueuse à 50%.

La suspension blanche résultante est mise au reflux sous forte agitation.

Au bout d'environ 30 minutes de reflux, on obtient une solution jaune vif limpide.

On introduit un fort balayage d'argon dans le ballon, de façon à éliminer l'eau. La solution est ainsi progressivement concentrée, jusqu'à l'obtention d'un matériau solide dur et cassant. On ajoute alors sous argon 280ml d'huile de silicone, le milieu réactionnel hétérogène ainsi obtenu est mis en chauffe à 145°C sous balayage d'argon pendant 24h. Aucune solubilisation/évolution de solide n'est observée pendant cette durée.

L'huile de silicone est éliminée par filtration. Le solide jaune est ensuite lavé avec IL de pentane sous forte agitation mécanique. On le disperse ensuite dans 1,51 de MeOH sous forte agitation. La suspension fluide ainsi obtenue est additionnée de 40ml d'HCl à 37%. Le précipité résultant est récupéré par filtration, puis séché à l'air.

Ce précipité est mis en suspension dans 1,51 d'acétonitrile sous forte agitation pendant une nuit. La suspension fluide résultante est filtrée, et le précipité obtenu recristallisé dans un mélange DMSO/acétone (1/9) et filtré à chaud. Après stockage du filtrat pendant 48h au réfrigérateur (1°C), on obtient 60g d'un précipité constitué de calixarènes géants.

Le filtrat correspondant est concentré à l'évaporateur rotatif (élimination de l'acétone), et additionné de 20ml d'éthanol.

La solution limpide ainsi obtenue est stockée au réfrigérateur (1°C) pendant 48h. La suspension obtenue est filtrée.

Le filtrat correspondant est concentré à l'évaporateur rotatif et précipité avec IL de méthanol. Après filtration, on obtient ainsi 25g d'un mélange contenant des « grands » calixarènes.

**Exemple 2 : Synthèse de grands calixarènes, avec traitement thermique dans le xylène**

Dans un ballon tricol de 2 1 équipé d'un agitateur mécanique, on introduit 104g de *p*-(benzyloxy)phénol, 140ml de formaldehyde en solution aqueuse à 37%, et 72ml de CsOH en solution aqueuse à 50%.

La suspension blanche résultante est mise au reflux sous forte agitation.

Au bout d'environ 30 minutes de reflux, on obtient une solution jaune vif limpide.

On introduit un fort balayage d'argon dans le ballon, de façon à éliminer l'eau. La solution est ainsi progressivement concentrée, jusqu'à l'obtention d'un matériau solide dur et cassant. On ajoute alors sous argon 200ml de xylène, le milieu réactionnel hétérogène ainsi obtenu est mis au reflux à 145°C sans agitation pendant 5h.

Le solide jaune obtenu est ensuite neutralisé avec un mélange de 500ml de THF et 50ml d'HCl à 37% sous forte agitation pendant une journée.

La suspension obtenue est évaporée à sec, puis lavée avec 1,2L de MeOH sous forte agitation pendant une nuit, puis filtrée. Le précipité obtenu est mis en suspension dans 1,51 d'acétonitrile sous forte agitation pendant une nuit. La suspension fluide résultante est filtrée, et le précipité obtenu recristallisé dans un mélange DMSO/acétone (175ml/2L) et filtré à chaud. Après stockage du filtrat pendant 48h au réfrigérateur (1°C), on obtient 20g d'un précipité constitué de calixarènes géants.

Le filtrat correspondant est concentré à l'évaporateur rotatif (élimination de l'acétone), et additionné de 20ml d'éthanol.

La solution limpide ainsi obtenue est stockée au réfrigérateur (1°C) pendant 48h. La suspension obtenue est filtrée.

Le filtrat correspondant est concentré à l'évaporateur rotatif et précipité avec IL de méthanol. Après filtration, on obtient ainsi 56g d'un mélange comportant des « grands » calixarènes.

**Exemple 3 : Synthèse de grands calixarènes, avec traitement thermique dans le xylène**

Dans un ballon tricol de 2 1 équipé d'un agitateur mécanique, on introduit 104g de *p*-(benzyloxy)phénol, 140ml de formaldehyde en solution aqueuse à 37%, et 50ml de RbOH en solution aqueuse à 50%.

La suspension blanche résultante est mise au reflux sous forte agitation.

Au bout d'environ 30 minutes de reflux, on obtient une solution jaune vif limpide.

On introduit un fort balayage d'argon dans le ballon, de façon à éliminer l'eau. La solution est ainsi progressivement concentrée, jusqu'à l'obtention d'un matériau solide dur et cassant. On ajoute alors sous argon 200ml de xylène, le milieu réactionnel hétérogène ainsi obtenu est mis au reflux à 145°C sans agitation pendant 7h30.

Le solide jaune obtenu est ensuite neutralisé avec un mélange de 500ml de THF et 50ml d'HCl à 37% sous forte agitation pendant une journée.

La suspension obtenue est évaporée à sec, puis lavée avec 2L de MeOH sous forte agitation pendant une nuit, puis filtrée. Le précipité obtenu est mis en suspension dans IL d'acétonitrile sous forte agitation pendant une nuit. La suspension fluide résultante est filtrée, et le précipité obtenu recristallisé dans un mélange DMSO/acétone (200ml/2L) et filtré à chaud. Après stockage du filtrat pendant 48h au réfrigérateur (1°C), on obtient 20g d'un précipité constitué de calixarènes géants.

Le filtrat correspondant est concentré à l'évaporateur rotatif (élimination de l'acétone), et additionné de 20ml d'éthanol.

La solution limpide ainsi obtenue est stockée au réfrigérateur (1°C) pendant 24h. La suspension obtenue est filtrée.

Le filtrat correspondant est concentré à l'évaporateur rotatif et précipité avec IL de méthanol. Après filtration, on obtient ainsi 30g d'un mélange comportant des « grands » calixarènes.

**Exemple 4 : Synthèse d'un polymère en étoile dérivé d'un grand calixarène**

***Synthèse de l'amorceur***

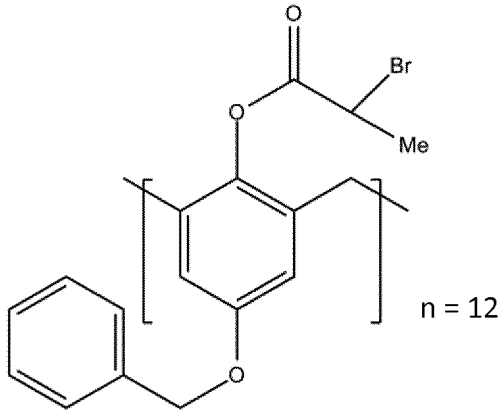

Un grand calixarène, ici le calix[12]arène (500mg, 0,0002mol), est introduit dans un tube de Schlenk. Sous argon, sont ensuite ajoutés 5 mL de DMF et 1,5 mL de $Et_3N$. Le système est plongé dans un bain de glace. Il est introduit 300 $\mu$L de bromure de bromopropionyle. La solution est agitée pendant 1h. Une précipitation est réalisée dans 50 mL MeOH. On filtre sous vide. L'amorceur est solubilisé dans quelques millilitres de THF. Puis on réalise une deuxième précipitation avec 50mL de MeOH. On filtre sous vide. Le produit est séché au dessiccateur.
Rendement = 60%

RMN 1H (350MHz, CDCl3) $\delta_{ppm}$: 7,1-7,6 (m, 60H, -$OCH_2$-*Ar*), 6,8-6,4 (m, 24H, Ar), 5,1-4,7 (m, 24H, -O$CH_2$-Ar-), 4-3,5 (m, 24H, -Ar(phenol)-$CH_2$-), 3-1,5 (m, 24H, $CH_3$-*CH*(COR)-Br), 2-1,5 (m, 36H, $CH_3$-CH(COR)-Br).

***Synthèse du polymère en étoile par polymérisation radicalaire par transfert d'atomes (ATRP)***

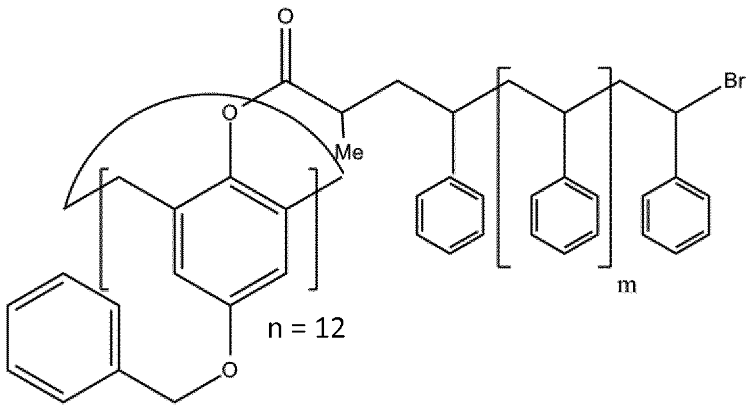

Dans un shlenk sous argon, il est introduit 25mg, (0,00017mol) de CuBr; la bipyridine (80mg, 0,00052mol) ; l'amorceur précédemment obtenu (60mg, 0,00017mol) et 2mL de styrène. Le milieu réactionnel est dégazé par 3 cycles de congélation-décongélation. Le shlenk est plongé dans un bain d'huile chauffé à 100°C pendant un temps donné. Le mélange est solubilisé dans du THF. Le mélange est filtré sur une colonne d'$Al_2O_3$ basique. Le polymère est précipité dans du MeOH $(\text{avec un rapport} = \frac{THF}{MeOH} = 10)$. Le polymère est filtré sous vide et rincé avec du MeOH. Le produit est séché au dessicateur.
Rendement = 50%.
RMN$^1$H (360MHz, CDCl3) $\delta_{ppm}$: 7,1-7,6 (m, 5H, -$OCH_2$-*Ar*), 7,2-6,9 (m, 5H, $Ar_{styrène}$), 7,2-6,9 (m, 1H, -$CH_2$-*CH*(Ar)-$CH_2$-),

6,8-6,4 (m, 2H, Ar), 2,1-1,6 (m, 2H, -CH-$CH_2$-CH-), 2,1-1,6 (m, 1H, $HO_2C$-*CH*($CH_3$)-$CH_2$-), 1,6-1,3 (m, 3H, $CH_3$-CH-)

Les polymères en étoile dérivés du calix[12]arène présentent un comportement hydrodynamique spécifique type « sphère dure », et forment donc en solution (THF) des nanoobjets bien définis, potentiellement intéressants pour l'encapsulation et la vectorisation.

## Revendications

**1.** Procédé de préparation :

- d'un grand *p*-(R)calixarène choisi parmi un *p*-(R)calix[9]arène, un *p*-(R)calix[10] arène, un *p*-(R)calix[11]arène, un *p*-(R)calix[12]arène, un *p*-(R)calix[13]arène, un *p*-(R)calix[14]arène, un *p*-(R)calix[15] arène, un *p*-(R)calix[16] arène, un *p*-(R)calix[17]arène, un *p*-(R)calix[18]arène, un *p*-(R)calix[19]arène, un *p*-(R)calix[20]arène, ou
- d'un mélange (a) d'au moins deux desdits grands *p*-(R)calixarènes dans lequel lesdits au moins deux grands *p*-(R)calixarènes sont présents dans ledit mélange à raison d'au moins 5% molaire chacun,

comprenant une étape de mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I) suivante :

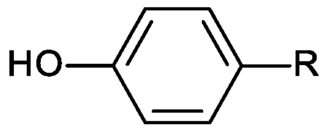

(I)

dans laquelle R est choisi parmi :

- un groupe benzyle éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyle en $C_1$-$C_{20}$ ramifié ou linéaire, à l'exclusion du groupe *t*-butyle,
- un groupe benzyloxy éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyloxy en $C_1$-$C_{20}$ ramifié ou linéaire,
- un groupe O-PEG 1 à 10 ramifié ou linéaire, dont le groupe terminal -OH est alkylé par un alkyle $C_1$-$C_{20}$ ramifié ou linéaire,
- un groupe benzylthioéther -S-$CH_2$-Ph éventuellement substitué en ortho et/ou para et/ou méta par un ou plusieurs substituants choisis parmi un halogène, SR' et OR', $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkylthioéther en $C_1$-$C_{20}$ ramifié ou linéaire, de formule -S-($C_1$-$C_{20}$ alkyle),
- un groupe -$NR_aR_b$, $R_a$ et $R_b$ représentant indépendamment l'un de l'autre un alkyle $C_1$-$C_{20}$ ramifié ou linéaire,
- un groupe dibenzylamine de formule -N(benzyle)$_2$, dans lequel les deux groupes benzyle sont, indépendamment l'un de l'autre, éventuellement substitués en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,

et une source de formaldéhyde aqueux,
en l'absence de solvant organique, constituant ainsi un milieu réactionnel initial,
ladite base étant à une concentration totale comprise d'environ 0,01 à 1 équivalent, notamment comprise d'environ 0,1 équivalent à environ 0,4 équivalent, en particulier égale à 0,15 ou 0,4 équivalent ou 1 équivalent, par rapport audit au moins un phénol substitué en position 4 de formule (I),

ledit milieu réactionnel initial étant chauffé à une température comprise de 100 à 165°C, pour obtenir un milieu réactionnel chauffé,
et comprenant, éventuellement, après ledit chauffage, une étape de traitement thermique supplémentaire dudit milieu réactionnel chauffé, en présence de moyens de transmission de chaleur, notamment un four ou un liquide chauffé,
pour obtenir un mélange (b), comprenant ledit grand *p*-(R)calixarène ou ledit mélange (a) d'au moins deux desdits grands *p*-(R)calixarènes, ledit mélange (b) comprenant de plus un mélange (c) de *p*-(R)calixarènes géants, dont la taille est supérieure à 20,
ledit procédé comprenant en outre, après ledit chauffage et l'éventuel traitement thermique supplémentaire, une étape de purification dudit mélange (b), afin d'éliminer ledit mélange (c) de *p*-(R)calixarènes géants, et d'obtenir ledit grand *p*-(R)calixarène ou ledit mélange (a) d'au moins deux desdits grands *p*-(R)calixarènes, dépourvu dudit mélange (c) de *p*-(R)calixarènes géants.

**2.** Procédé selon la revendication 1, de préparation :

- d'un grand *p*-(R)calixarène choisi parmi un *p*-(R)calix[9]arène, un *p*-(R)calix[10] arène, un *p*-(R)calix[11]arène, un *p*-(R)calix[12]arène, ou
- d'un mélange (a) d'au moins deux desdits grands *p*-(R)calixarènes dans lequel lesdits au moins deux grands *p*-(R)calixarènes sont présents dans ledit mélange à raison d'au moins 5% molaire chacun.

**3.** Procédé selon la revendication 1, de préparation :

- d'un grand *p*-($R_1$-oxy)calixarène choisi parmi un *p*-($R_1$-oxy)calix[9]arène, un *p*-($R_1$-oxy)calix[10]arène, un *p*-($R_1$-oxy)calix[11]arène, un *p*-($R_1$-oxy)calix[12]arène, un *p*-($R_1$-oxy)calix[13]arène, un *p*-($R_1$-oxy)calix[14]arène, un *p*-($R_1$-oxy)calix[15]arène, un *p*-($R_1$-oxy)calix[16]arène, un *p*-($R_1$-oxy)calix[17]arène, un *p*-($R_1$-oxy)calix[18]arène, un *p*-($R_1$-oxy)calix[19]arène, un *p*-($R_1$-oxy)calix[20]arène, ou
- d'un mélange d'au moins deux desdits grands *p*-($R_1$-oxy)calixarènes dans lequel lesdits au moins deux grands *p*-($R_1$-oxy)calixarènes sont présents dans ledit mélange à raison d'au moins 5% molaire chacun,

comprenant une étape de mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 est de formule (Ia) suivante :

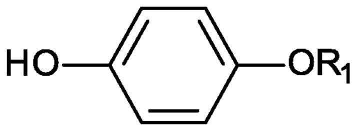

(Ia)

dans laquelle $R_1$ est choisi parmi :

- un groupe benzyle éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyle en $C_1$-$C_{20}$ ramifié ou linéaire,
- un PEG 1 à 10 ramifié ou linéaire, dont le groupe terminal -OH est alkylé par un alkyle $C_1$-$C_{20}$ ramifié ou linéaire,

et une source de formaldéhyde aqueux,
en l'absence de solvant organique, constituant ainsi un milieu réactionnel initial, ladite base étant à une concentration totale comprise d'environ 0,01 à 1 équivalent, notamment comprise d'environ 0,1 équivalent à environ 0,4 équivalent, en particulier égale à 0,15 ou 0,4 équivalent ou 1 équivalent, par rapport audit au moins un phénol substitué en position 4 de formule (I),
ledit milieu réactionnel initial étant chauffé à une température comprise de 100 à 165°C, pour obtenir un milieu réactionnel chauffé,
et comprenant, éventuellement, après ledit chauffage, une étape de traitement thermique supplémentaire dudit milieu réactionnel chauffé, en présence de moyens de transmission de chaleur, notamment un four ou un liquide

chauffé,
pour obtenir un mélange (b), comprenant ledit grand *p*-($R_1$-oxy)calixarène ou ledit mélange (a) d'au moins deux desdits grands *p*-($R_1$-oxy)calixarènes, ledit mélange (b) comprenant de plus un mélange (c) de *p*-($R_1$-oxy)calixarènes géants, dont la taille est supérieure à 20,
ledit procédé comprenant en outre, après ledit chauffage et l'éventuel traitement thermique supplémentaire, une étape de purification dudit mélange (b), afin d'éliminer ledit mélange (c) de *p*-($R_1$-oxy)calixarènes géants, et d'obtenir ledit grand *p*-($R_1$-oxy)calixarène ou ledit mélange (a) d'au moins deux desdits grands *p*-($R_1$-oxy)calixarènes, dépourvu dudit mélange (c) de *p*-($R_1$-oxy)calixarènes géants.

**4.** Procédé selon l'une quelconque des revendications 1 à 3, de préparation :

- d'un grand *p*-($R_1$-oxy)calixarène choisi parmi un *p*-($R_1$-oxy)calix[9]arène, un *p*-($R_1$-oxy)calix[10]arène, un *p*-($R_1$-oxy)calix[11]arène, un *p*-($R_1$-oxy)calix[12]arène, ou
- d'un mélange d'au moins deux desdits grands *p*-($R_1$-oxy)calixarènes dans lequel lesdits au moins deux grands *p*-($R_1$-oxy)calixarènes sont présents dans ledit mélange à raison d'au moins 5% molaire chacun.

**5.** Procédé selon l'une quelconque des revendications 1 à 4, dans lequel l'eau présente dans le milieu réactionnel initial ainsi que celle produite lors de la réaction sont éliminées dudit milieu réactionnel durant ledit chauffage et un précurseur solide sous forme de résine dure cassante, éventuellement isolé, est obtenu,
*notamment dans lequel ledit précurseur solide sous forme de résine dure cassante est isolé du susdit milieu réactionnel et dépourvu de traitement thermique supplémentaire,
*notamment dans lequel ledit précurseur solide sous forme de résine dure cassante, contenu dans ledit milieu réactionnel, est mis en présence de moyens de transmission de chaleur sous la forme d'un four, ou d'un liquide chauffé dans lequel ledit précurseur solide sous forme de résine dure cassante, est très peu soluble ou totalement insoluble, notamment dans le toluène, le xylène, un alcane linéaire ou ramifié de point d'ébullition supérieur à 50°C, notamment supérieur à 100°C, en particulier l'octane, ou une huile de silicone, et est soumis à un traitement thermique supplémentaire,
*ledit procédé comprenant notamment les étapes suivantes :

a. mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I), en particulier de formule (Ia),
avec du formaldéhyde aqueux, pour constituer un milieu réactionnel initial et chauffage dudit milieu réactionnel initial durant un temps compris de 20 minutes à 2 heures, en particulier d'environ 30 minutes à environ 1 heure, tout en éliminant éventuellement l'eau présente dans le milieu réactionnel initial ainsi que celle formée,
b. poursuite du chauffage durant 1 à 3 heures dudit milieu réactionnel tout en éliminant l'eau présente dans le milieu réactionnel ainsi que celle formée pour obtenir un milieu réactionnel contenant un précurseur solide, sous forme de résine dure cassante,
c. éventuellement :

◦ chauffage dudit milieu réactionnel ou dudit précurseur solide à l'aide de moyens de transmission de chaleur sous la forme d'un four, durant 3 à 24 heures,
◦ ou addition audit milieu réactionnel ou audit précurseur solide sous forme de résine dure cassante d'un liquide dans lequel ledit précurseur solide sous forme de résine dure cassante est très peu soluble ou totalement insoluble, notamment dans le toluène, le xylène, un alcane linéaire ou ramifié de point d'ébullition supérieur à 50°C, notamment supérieur à 100°C, en particulier l'octane ou une huile de silicone, pour obtenir un milieu réactionnel contenant un solide sous forme de résine dure cassante, et chauffage dudit milieu réactionnel contenant un solide sous forme de résine dure cassante, durant 3 à 24 heures, avec ou sans agitation,

pour obtenir un mélange (b) comprenant ledit grand *p*-(R)calixarène ou ledit mélange (a) d'au moins deux desdits grands *p*-(R)calixarènes, ledit mélange (b) comprenant de plus un mélange (c) de *p*-(R)calixarènes géants, dont la taille est supérieure à 20,
d. éventuellement, après neutralisation de la base, au moins un lavage dudit mélange (b) par un solvant polaire, en particulier le méthanol ou l'acétonitrile, pour obtenir ledit mélange (b), sous forme neutralisée,
e. purification dudit mélange (b) éventuellement neutralisé, afin d'éliminer ledit mélange (c) de *p*-(R)calixarènes géants, et d'obtenir ledit grand *p*-(R)calixarène ou ledit mélange (a) d'au moins deux desdits grands *p*-(R)calixarènes, dépourvu dudit mélange (c) de *p*-(R)calixarènes géants,

f. éventuellement, purification du mélange (a) par GPC, en séparant différentes fractions en fonction de leur temps d'élution, pour obtenir ledit grand *p*-(R)calixarène.

**6.** Procédé selon l'une quelconque des revendications 1 à 5, comprenant les étapes suivantes :

a. mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I), en particulier de formule (Ia),
avec du formaldéhyde aqueux, pour constituer un milieu réactionnel initial et chauffage dudit milieu réactionnel initial durant un temps compris de 20 minutes à 2 heures, en particulier d'environ 30 minutes à environ 1 heure, tout en éliminant éventuellement l'eau présente dans le milieu réactionnel initial ainsi que celle formée,
b. poursuite du chauffage durant 1 à 3 heures dudit milieu réactionnel tout en éliminant l'eau présente dans le milieu réactionnel ainsi que celle formée pour obtenir un milieu réactionnel contenant un précurseur solide, sous forme de résine dure cassante,
c. chauffage dudit milieu réactionnel ou dudit précurseur solide à l'aide de moyens de transmission de chaleur sous la forme d'un four, durant 3 à 24 heures,
ou addition audit milieu réactionnel ou audit précurseur solide sous forme de résine dure cassante d'un liquide dans lequel ledit précurseur solide sous forme de résine dure cassante est très peu soluble ou totalement insoluble, notamment dans le toluène, le xylène, un alcane linéaire ou ramifié de point d'ébullition supérieur à 50°C, notamment supérieur à 100°C, en particulier l'octane ou une huile de silicone, pour obtenir un milieu réactionnel contenant un solide sous forme de résine dure cassante, et chauffage dudit milieu réactionnel contenant un solide sous forme de résine dure cassante, durant 3 à 24 heures, avec ou sans agitation, pour obtenir un mélange (b) comprenant ledit grand *p*-(R)calixarène ou ledit mélange (a) d'au moins deux desdits grands *p*-(R)calixarènes, ledit mélange (b) comprenant de plus un mélange (c) de *p*-(R)calixarènes géants, dont la taille est supérieure à 20,
d. éventuellement, après neutralisation de la base, au moins un lavage dudit mélange (b) par un solvant polaire, en particulier le méthanol ou l'acétonitrile, pour obtenir ledit mélange (b), sous forme neutralisée,
e. purification dudit mélange (b) éventuellement neutralisé, afin d'éliminer ledit mélange (c) de *p*-(R)calixarènes géants, et d'obtenir ledit grand *p*-(R)calixarène ou ledit mélange (a) d'au moins deux desdits grands *p*-(R)calixarènes, dépourvu dudit mélange (c) de *p*-(R)calixarènes géants,
f. éventuellement, purification du mélange (a) par GPC, en séparant différentes fractions en fonction de leur temps d'élution, pour obtenir ledit grand p-(R)calixarène.

**7.** Procédé selon l'une quelconque des revendications 1 à 3, dans lequel l'eau présente dans le milieu réactionnel initial ainsi que celle produite lors de la réaction sont conservées dans le milieu réactionnel durant ledit chauffage et un précurseur solide sous forme de précipité, éventuellement isolé, est obtenu,
*notamment dans lequel ledit précurseur solide sous forme de précipité est isolé du susdit milieu réactionnel et dépourvu de traitement thermique supplémentaire,
*ledit procédé comprenant notamment les étapes suivantes :

a. mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I), en particulier de formule (Ia),
avec du formaldéhyde aqueux, pour constituer un milieu réactionnel initial et chauffage dudit milieu réactionnel initial durant un temps compris de 20 minutes à 5 heures, en particulier d'environ 30 minutes à environ 2 heures, sans élimination de l'eau présente dans le milieu réactionnel initial ainsi que de celle formée, pour obtenir un milieu réactionnel contenant un précurseur solide, sous forme de précipité,
b. éventuellement, addition audit milieu réactionnel ou audit précurseur solide sous forme de précipité d'un liquide dans lequel ledit précurseur solide sous forme de précipité est très peu soluble ou totalement insoluble, notamment dans le toluène le xylène, un alcane linéaire ou ramifié de point d'ébullition supérieur à 50°C, notamment supérieur à 100°C, en particulier l'octane ou une huile de silicone, pour obtenir un milieu réactionnel contenant un solide sous forme de précipité, et chauffage dudit milieu réactionnel contenant un solide sous forme de précipité, durant 3 à 24 heures, avec ou sans agitation,
pour obtenir un mélange (b) comprenant ledit grand *p*-(R)calixarène ou ledit mélange (a) d'au moins deux desdits grands *p*-(R)calixarènes, ledit mélange (b) comprenant de plus un mélange (c) de *p*-(R)calixarènes géants, dont la taille est supérieure à 20,
c. éventuellement, après neutralisation de la base, au moins un lavage dudit mélange (b) par un solvant polaire, en particulier le méthanol ou l'acétonitrile, pour obtenir ledit mélange (b), sous forme neutralisée,

d. purification dudit mélange (b) éventuellement neutralisé, afin d'éliminer ledit mélange (c) de *p*-(R)calixarènes géants, et d'obtenir ledit grand *p*-(R)calixarène ou ledit mélange (a) d'au moins deux desdits grands *p*-(R)calixarènes, dépourvu dudit mélange (c) de *p*-(R)calixarènes géants,
e. éventuellement, purification du mélange (a) par GPC, en séparant différentes fractions en fonction de leur temps d'élution, pour obtenir ledit grand *p*-(R)calixarène.

**8.** Procédé selon la revendication 7, dans lequel l'eau présente dans le milieu réactionnel initial ainsi que celle produite lors de la réaction sont conservées dans ledit milieu réactionnel durant ledit chauffage et un précurseur solide sous forme de précipité, éventuellement isolé, est obtenu,
*notamment dans lequel ledit précurseur solide sous forme de précipité est isolé du susdit milieu réactionnel et dépourvu de traitement thermique supplémentaire,
*ledit procédé comprenant notamment les étapes suivantes :

a. mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I), en particulier de formule (Ia),
avec du formaldéhyde aqueux, pour constituer un milieu réactionnel initial et chauffage dudit milieu réactionnel initial durant un temps compris de 20 minutes à 5 heures, en particulier d'environ 30 minutes à environ 2 heures, sans élimination de l'eau présente dans le milieu réactionnel initial ainsi que de celle formée, pour obtenir un milieu réactionnel contenant un précurseur solide, sous forme de précipité,
b. addition audit milieu réactionnel ou audit précurseur solide sous forme de précipité d'un liquide dans lequel ledit précurseur solide sous forme de précipité est très peu soluble ou totalement insoluble, notamment dans le toluène, le xylène, un alcane linéaire ou ramifié de point d'ébullition supérieur à 50°C, notamment supérieur à 100°C, en particulier l'octane ou une huile de silicone, pour obtenir un milieu réactionnel contenant un solide sous forme de précipité, et chauffage dudit milieu réactionnel contenant un solide sous forme de précipité, durant 3 à 24 heures, avec ou sans agitation,
pour obtenir un mélange (b) comprenant ledit grand *p*-(R)calixarène ou ledit mélange (a) d'au moins deux desdits grands *p*-(R)calixarènes, ledit mélange (b) comprenant de plus un mélange (c) de *p*-(R)calixarènes géants, dont la taille est supérieure à 20,
c. éventuellement, après neutralisation de la base, au moins un lavage dudit mélange (b) par un solvant polaire, en particulier le méthanol ou l'acétonitrile, pour obtenir ledit mélange (b), sous forme neutralisée,
d. purification dudit mélange (b) éventuellement neutralisé, afin d'éliminer ledit mélange (c) de *p*-(R)calixarènes géants, et d'obtenir ledit grand *p*-(R)calixarène ou ledit mélange (a) d'au moins deux desdits grands *p*-(R)calixarènes, dépourvu dudit mélange (c) de *p*-(R)calixarènes géants,
e. éventuellement, purification du mélange (a) par GPC, en séparant différentes fractions en fonction de leur temps d'élution, pour obtenir ledit grand *p*-(R)calixarène.

**9.** Procédé selon l'une quelconque des revendications 1 à 8, dans lequel ladite purification dudit mélange (b) comprend une étape de recristallisation dudit mélange (b) pour obtenir à l'issue de l'étape de recristallisation un précipité et un filtrat, ledit filtrat comprenant ledit grand *p*-(R)calixarène ou ledit mélange (a) d'au moins deux desdits grands *p*-(R)calixarènes, dépourvu dudit mélange (c) de *p*-(R)calixarènes géants, et le précipité comprenant ledit mélange (c) de *p*-(R)calixarènes géants, dont la taille est supérieure à 20.

**10.** Procédé selon l'une quelconque des revendications 1 à 8, dans lequel ladite purification dudit mélange (b) comprend une étape de recristallisation à deux solvants dudit mélange (b) pour obtenir, après addition du deuxième solvant, un précipité et un filtrat, ledit filtrat comprenant ledit grand *p*-(R)calixarène ou ledit mélange (a) d'au moins deux desdits grands *p*-(R)calixarènes, et le mélange (c) de *p*-(R)calixarènes géants, et le précipité comprenant principalement du calix[8]arène.

**11.** Procédé selon l'une quelconque des revendications 1 à 8, dans lequel ladite purification dudit mélange (b) comprend les étapes suivantes :

- dissolution dudit mélange (b) dans le minimum d'un premier solvant, à chaud, pour obtenir une solution, à chaud, dudit mélange (b),
- ajout, à la solution, à chaud, dudit mélange (b), d'un deuxième solvant jusqu'à apparition d'un précipité, et filtration immédiate, pour obtenir un filtrat contenant les grands calixarènes et les calixarènes géants,
- après refroidissement du filtrat obtenu précédemment, filtration dudit précipité pour obtenir :

o un filtrat comprenant ledit grand *p*-(R)calixarène ou ledit mélange (a) d'au moins deux desdits grands *p*-(R)calixarènes, dépourvu dudit mélange (c) de *p*-(R)calixarènes géants, et
o le précipité isolé, comprenant ledit mélange (c) de *p*-(R)calixarènes géants, dont la taille est supérieure à 20.

**12.** Procédé selon l'une quelconque des revendications 1 à 8, dans lequel ladite purification dudit mélange (b) comprend les étapes suivantes :

- dissolution dudit mélange (b) dans le minimum d'un premier solvant, à chaud, pour obtenir une solution, à chaud, dudit mélange (b),
- ajout, à la solution, à chaud, dudit mélange (b), d'un deuxième solvant jusqu'à apparition d'un premier précipité,
- filtration à chaud dudit premier précipité pour obtenir un filtrat,
- refroidissement du filtrat pour obtenir la formation d'un deuxième précipité,
- filtration dudit deuxième précipité pour obtenir :

◦ un filtrat comprenant ledit grand *p*-(R)calixarène ou ledit mélange (a) d'au moins deux desdits grands *p*-(R)calixarènes, dépourvu dudit mélange (c) de *p*-(R)calixarènes géants, et
◦ le précipité isolé, comprenant ledit mélange (c) de *p*-(R)calixarènes géants, dont la taille est supérieure à 20.

**13.** Procédé selon l'une quelconque des revendications 1 à 8, dans lequel ladite purification dudit mélange (b) comprend les étapes suivantes :

- dissolution dudit mélange (b) dans le minimum de DMSO, à une température comprise d'environ 80°C à environ 160°C, en particulier d'environ 100°C à environ 140°C, plus particulièrement à une température d'environ 120°C, pour obtenir une solution, à chaud, dudit mélange (b),
- ajout, à la solution, à chaud, dudit mélange (b), d'un deuxième solvant moyennement polaire, en particulier l'acétone, jusqu'à apparition d'un premier précipité,
- filtration à chaud dudit premier précipité pour obtenir un filtrat,
- refroidissement du filtrat à environ 0°C pour obtenir la formation d'un deuxième précipité,
- filtration dudit deuxième précipité pour obtenir :

◦ un filtrat comprenant ledit grand *p*-(R)calixarène ou ledit mélange (a) d'au moins deux desdits grands *p*-(R)calixarènes, dépourvu dudit mélange (c) de *p*-(R)calixarènes géants, et
◦ le précipité isolé, comprenant ledit mélange (c) de *p*-(R)calixarènes géants, dont la taille est supérieure à 20,

- concentration dudit filtrat pour obtenir ledit grand *p*-(R)calixarène ou ledit mélange (a) d'au moins deux desdits grands *p*-(R)calixarènes, dépourvu dudit mélange (c) de *p*-(R)calixarènes géants.

**14.** Procédé selon l'une quelconque des revendications 1 à 8, dans lequel ladite purification dudit mélange (b) comprend les étapes suivantes :

- dissolution dudit mélange (b) dans le minimum de DMSO, à une température comprise d'environ 80°C à environ 160°C, en particulier d'environ 100°C à environ 140°C, plus particulièrement à une température d'environ 120°C, pour obtenir une solution, à chaud, dudit mélange (b),
- ajout, à la solution, à chaud, dudit mélange (b), d'un deuxième solvant moyennement polaire, en particulier l'acétone, jusqu'à apparition d'un premier précipité,
- filtration à chaud dudit premier précipité pour obtenir un filtrat,
- refroidissement du filtrat à environ 0°C pour obtenir la formation d'un deuxième précipité,
- filtration dudit deuxième précipité pour obtenir :

◦ un filtrat comprenant ledit grand *p*-(R)calixarène ou ledit mélange (a) d'au moins deux desdits grands *p*-(R)calixarènes, dépourvu dudit mélange (c) de *p*-(R)calixarènes géants, et
◦ le précipité isolé, comprenant ledit mélange (c) de *p*-(R)calixarènes géants, dont la taille est supérieure à 20,

- concentration dudit filtrat pour obtenir un résidu dépourvu d'acétone,
- ajout audit résidu d'un solvant polaire, en particulier l'éthanol, notamment à température ambiante, pour obtenir une solution dudit résidu dans ledit solvant polaire,
- refroidissement de ladite solution dudit résidu, en particulier à environ 0°C, pour obtenir une suspension, ladite

suspension étant filtrée pour obtenir un filtrat, ledit filtrat étant concentré pour obtenir ledit grand *p*-(R)calixarène ou ledit mélange (a) d'au moins deux desdits grands *p*-(R)calixarènes, dépourvu dudit mélange (c) de *p*-(R)calixarènes géants.

## Patentansprüche

**1.** Verfahren zur Herstellung:

- eines großen *p*-(R)-Calixarens, ausgewählt aus einem *p*-(R)-Calix[9]aren, einem *p*-(R)-Calix[10]aren, einem *p*-(R)-Calix[11]aren, einem *p*-(R)-Calix[12]aren, einem *p*-(R)-Calix[13]aren, einem *p*-(R)-Calix[14]aren, einem *p*-(R)-Calix[15]aren, einem *p*-(R)-Calix[16]aren, einem *p*-(R)-Calix[17]aren, einem *p*-(R)-Calix[18]aren, einem *p*-(R)-Calix[19]aren, einem *p*-(R)-Calix[20]aren, oder
- einer Mischung (a) von mindestens zwei der genannten großen *p*-(R)-Calixarene, wobei die genannten mindestens zwei großen *p*-(R)-Calixarene in der Mischung in einer Menge von jeweils mindestens 5 Mol-% vorhanden sind,

umfassend einen Schritt des Inberührungbringens mindestens einer Base, die insbesondere aus Bariumhydroxid, Rubidiumhydroxid, Lithiumhydroxid, Cesiumhydroxid, Kaliumchlorid oder Natriumcarbonat ausgewählt ist, mit mindestens einem Phenol, das substituiert ist in Stellung 4 der folgenden Formel (I) :

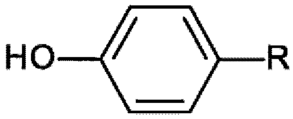

(I)

wobei R ausgewählt ist aus:

- einer Benzylgruppe, die gegebenenfalls in ortho und/oder para und/oder meta durch einen oder mehrere Substituenten substituiert ist, ausgewählt aus einem Halogen, SR' und OR', wobei R' eine lineare oder verzweigte $C_1$-$C_{20}$-Alkylkette, eine $C_3$-$C_{20}$-Cycloalkylgruppe, $NR_aR_b$, $PR_aR_b$, $P(O)R_aR_b$, $P(O)OR_aOR_b$ darstellt, wobei $R_a$ und $R_b$ unabhängig voneinander ein verzweigtes oder lineares $C_1$-$C_{20}$-Alkyl darstellen,
- einer verzweigten oder linearen $C_1$-$C_{20}$-Alkylgruppe, mit Ausnahme einer t-Butylgruppe,
- einer Benzyloxygruppe, die gegebenenfalls in ortho und/oder para und/oder meta durch einen oder mehrere Substituenten substituiert ist, ausgewählt aus einem Halogen, SR' und OR', wobei R' eine lineare oder verzweigte $C_1$-$C_{20}$-Alkylkette, eine $C_3$-$C_{20}$-Cycloalkylgruppe, $NR_aR_b$, $PR_aR_b$, $P(O)R_aR_b$, $P(O)OR_aOR_b$ darstellt, wobei $R_a$ und $R_b$ unabhängig voneinander ein verzweigtes oder lineares $C_1$-$C_{20}$-Alkyl darstellen,
- einer verzweigten oder linearen $C_1$-$C_{20}$-Alkyloxygruppe,
- einer verzweigten oder linearen Gruppe O-PEG 1 bis 10, deren Endgruppe -OH durch ein verzweigtes oder lineares $C_1$-$C_{20}$-Alkyl alkyliert ist,
- einer Benzylthioethergruppe $-S-CH_2-Ph$, die gegebenenfalls in ortho und/oder para und/oder meta durch einen oder mehrere Substituenten substituiert ist, ausgewählt aus einem Halogen, SR' und OR', $NR_aR_b$, $PR_aR_b$, $P(O)R_aR_b$, $P(O)OR_aOR_b$, wobei $R_a$ und $R_b$ unabhängig voneinander ein verzweigtes oder lineares $C_1$-$C_{20}$-Alkyl darstellen,
- einer verzweigten oder linearen $C_1$-$C_{20}$-Alkylthioethergruppe mit der Formel -S-($C_1$-$C_{20}$-Alkyl),
- einer Gruppe $-NR_aR_b$, wobei $R_a$ und $R_b$ unabhängig voneinander ein verzweigtes oder lineares $C_1$-$C_{20}$-Alkyl darstellen,
- einer Dibenzylamingruppe mit der Formel $-N(Benzyl)_2$, wobei die beiden Benzylgruppen unabhängig voneinander gegebenenfalls in ortho und/oder para und/oder meta durch einen oder mehrere Substituenten substituiert sind, ausgewählt aus einem Halogen, SR' und OR', wobei R' eine lineare oder verzweigte $C_1$-$C_{20}$-Alkylkette, eine $C_3$-$C_{20}$-Cycloalkylgruppe, $NR_aR_b$, $PR_aR_b$, $P(O)R_aR_b$, $P(O)OR_aOR_b$ darstellt, wobei $R_a$ und $R_b$ unabhängig voneinander ein verzweigtes oder lineares $C_1$-$C_{20}$-Alkyl darstellen

und einer Quelle von wässrigem Formaldehyd,
in Abwesenheit eines organischen Lösungsmittels, wobei so ein Anfangsreaktionsmedium gebildet wird,
wobei die genannte Base eine Gesamtkonzentration von ungefähr 0,01 Äquivalenten bis 1 Äquivalent, insbesondere von ungefähr 0,1 Äquivalenten bis ungefähr 0,4 Äquivalenten, insbesondere gleich 0,15 oder 0,4 Äquivalenten oder

1 Äquivalent aufweist, bezogen auf das genannte mindestens eine Phenol, das in Stellung 4 der Formel (I) substituiert ist,
wobei das Anfangsreaktionsmedium auf eine Temperatur von 100 bis 165 °C erhitzt wird, um ein erhitztes Reaktionsmedium zu erhalten,
und umfassend, gegebenenfalls nach dem Erhitzen, einen ergänzenden Schritt einer thermischen Behandlung des genannten erhitzten Reaktionsmediums in Anwesenheit von Wärmeübertragungsmitteln, insbesondere eines Ofens oder einer erhitzten Flüssigkeit,
um eine Mischung (b) zu erhalten, umfassend das genannte große *p*-(R)-Calixaren oder die genannte Mischung (a) von mindestens zwei der genannten großen p-(R)-Calixarene, wobei die genannte Mischung (b) ferner eine Mischung (c) von riesigen *p*-(R)-Calixarenen umfasst, deren Größe größer ist als 20,
wobei das Verfahren außerdem, nach dem genannten Erhitzen und der optionalen ergänzenden thermischen Behandlung, einen Schritt der Reinigung der genannten Mischung (b) umfasst, um die Mischung (c) von riesigen p-(R)-Calixarenen zu eliminieren, und um das genannte große p-(R)-Calixaren oder die genannte Mischung (a) von mindestens zwei der genannten großen p-(R)-Calixarene ohne die genannte Mischung (c) von riesigen *p*-(R)-Calixarenen zu erhalten.

**2.** Verfahren nach Anspruch 1, zur Herstellung:

- eines großen *p*-(R)-Calixarens, ausgewählt aus einem *p*-(R)-Calix[9]aren, einem *p*-(R)-Calix[10]aren, einem *p*-(R)-Calix[11]aren, einem *p*-(R)-Calix[12]aren, oder
- einer Mischung (a) von mindestens zwei der genannten großen *p*-(R)-Calixarene, wobei die genannten mindestens zwei großen *p*-(R)-Calixarene in der Mischung in einer Menge von jeweils mindestens 5 Mol-% vorhanden sind.

**3.** Verfahren nach Anspruch 1, zur Herstellung:

- eines großen *p*-($R_1$-Oxy)-calixarens, ausgewählt aus einem *p*-($R_1$-Oxy)-calix[9]aren, einem *p*-($R_1$-Oxy)-calix[10]aren, einem *p*-($R_1$-Oxy) - calix[11]aren, einem *p*-($R_1$-Oxy)-calix[12]aren, einem *p*-($R_1$-Oxy)-calix[13]aren, einem *p*-($R_1$-Oxy)-calix[14]aren, einem *p*-($R_1$-Oxy)-calix[15]aren, einem *p*-($R_1$-Oxy)-calix[16]aren, einem *p*-($R_1$-Oxy)-calix[17]aren, einem *p*-($R_1$-Oxy)-calix[18]aren, einem *p*-($R_1$-Oxy)-calix[19]aren, einem *p*-($R_1$-Oxy)-calix[20]aren, oder
- einer Mischung von mindestens zwei der genannten großen *p*-($R_1$-Oxy)-calixarene, wobei die genannten mindestens zwei großen *p*-($R_1$-Oxy)-calixarene in der Mischung in einer Menge von jeweils mindestens 5 Mol-% vorhanden sind,

umfassend einen Schritt des Inberührungbringens mindestens einer Base, welche insbesondere aus Bariumhydroxid, Rubidiumhydroxid, Lithiumhydroxid, Cesiumhydroxid, Kaliumchlorid oder Natriumcarbonat ausgewählt ist, mit mindestens einem Phenol, das substituiert ist in Stellung 4 der folgenden Formel (Ia) :

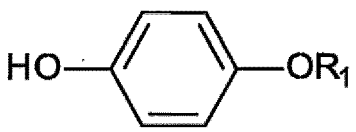

(Ia)

wobei $R_1$ ausgewählt ist aus:

- einer Benzylgruppe, die gegebenenfalls in ortho und/oder para und/oder meta durch einen oder mehrere Substituenten substituiert ist, ausgewählt aus einem Halogen, SR' und OR', wobei R' eine lineare oder verzweigte $C_1$-$C_{20}$-Alkylkette, eine $C_3$-$C_{20}$-Cycloalkylgruppe, $NR_aR_b$, $PR_aR_b$, $P(O)R_aR_b$, $P(O)OR_aOR_b$ darstellt, wobei $R_a$ und $R_b$ unabhängig voneinander ein verzweigtes oder lineares $C_1$-$C_{20}$-Alkyl darstellen,
- einer verzweigten oder linearen $C_1$-$C_{20}$-Alkylgruppe,
- einem verzweigten oder linearen PEG 1 bis 10, dessen Endgruppe -OH durch ein verzweigtes oder lineares $C_1$-$C_{20}$-Alkyl alkyliert ist,

und einer Quelle von wässrigem Formaldehyd,
in Abwesenheit eines organischen Lösungsmittels, wobei so ein Anfangsreaktionsmedium gebildet wird,

wobei die genannte Base eine Gesamtkonzentration von ungefähr 0,01 Äquivalenten bis 1 Äquivalent, insbesondere von ungefähr 0,1 Äquivalenten bis ungefähr 0,4 Äquivalenten, insbesondere gleich 0,15 oder 0,4 Äquivalenten oder 1 Äquivalent aufweist, bezogen auf das genannte mindestens eine Phenol, das in Stellung 4 der Formel (I) substituiert ist,
wobei das genannte Anfangsreaktionsmedium auf eine Temperatur von 100 bis 165 °C erhitzt wird, um ein erhitztes Reaktionsmedium zu erhalten,
und umfassend, gegebenenfalls nach dem genannten Erhitzen, einen ergänzenden Schritt einer thermischen Behandlung des genannten erhitzten Reaktionsmediums in Anwesenheit von Wärmeübertragungsmitteln, insbesondere eines Ofens oder einer erhitzten Flüssigkeit,
um eine Mischung (b) zu erhalten, umfassend das genannte große *p*-($R_1$-Oxy)-calixaren oder die genannte Mischung (a) von mindestens zwei der genannten großen *p*-($R_1$-Oxy)-calixarene, wobei die genannte Mischung (b) ferner eine Mischung (c) von riesigen *p*-($R_i$-Oxy)-calixarenen umfasst, deren Größe größer ist als 20, wobei das genannte Verfahren außerdem, nach dem genannten Erhitzen und der optionalen ergänzenden thermischen Behandlung, einen Schritt der Reinigung der genannten Mischung (b) umfasst, um die genannte Mischung (c) von riesigen *p*-($R_1$-Oxy)-calixarenen zu eliminieren, und um das genannte große *p*-($R_1$-Oxy)-calixaren oder die genannte Mischung (a) von mindestens zwei der genannten großen *p*-($R_1$-Oxy)-calixarene ohne die genannte Mischung (c) von riesigen *p*-($R_1$-Oxy)-calixarenen zu erhalten.

**4.** Verfahren nach einem der Ansprüche 1 bis 3, zur Herstellung:

- eines großen *p*-($R_1$-Oxy)-calixarens, ausgewählt aus einem *p*-($R_1$-Oxy)-calix[9]aren, einem *p*-($R_1$-Oxy)-calix[10]aren, einem *p*-($R_1$-Oxy)-calix[11]aren, einem *p*-($_{1i}$-Oxy)-calix[12]aren, oder
- einer Mischung von mindestens zwei der genannten großen *p*-($R_1$-Oxy)-calixarene, wobei die genannten mindestens zwei großen *p*-($R_1$-Oxy)-calixarene in der genannten Mischung in einer Menge von jeweils mindestens 5 Mol-% vorhanden sind.

**5.** Verfahren nach einem der Ansprüche 1 bis 4, wobei das Wasser, das in dem Anfangsreaktionsmedium vorhanden ist, sowie jenes, das bei der Reaktion erzeugt wird, aus dem genannten Reaktionsmedium während des genannten Erhitzens entfernt wird, und ein fester Vorläufer in der Form eines brüchigen harten Harzes, das gegebenenfalls isoliert wird, erhalten wird,

* wobei insbesondere der genannte feste Vorläufer in der Form eines brüchigen harten Harzes aus dem genannten Reaktionsmedium isoliert wird und keiner ergänzenden thermischen Behandlung unterzogen wird,
* wobei insbesondere der genannte feste Vorläufer in der Form eines brüchigen harten Harzes, das in dem genannten Reaktionsmedium enthalten ist, mit Wärmeübertragungsmitteln in der Form eines Ofens oder einer erhitzten Flüssigkeit in Kontakt gebracht wird, in welcher der genannte feste Vorläufer in der Form eines brüchigen harten Harzes sehr wenig löslich oder vollständig unlöslich ist, insbesondere in Toluol, Xylol, einem linearen oder verzweigten Alkan mit einem Siedepunkt von mehr als 50 °C, insbesondere mehr als 100 °C, insbesondere Octan, oder einem Silikonöl, und einer ergänzenden thermischen Behandlung unterzogen wird,
* wobei das genannte Verfahren insbesondere die folgenden Schritte umfasst:

a. Inberührungbringen mindestens einer Base, die insbesondere aus Bariumhydroxid, Rubidiumhydroxid, Lithiumhydroxid, Cesiumhydroxid, Kaliumchlorid oder Natriumcarbonat ausgewählt ist, mit mindestens einem Phenol, das in Stellung 4 der Formel (I), insbesondere der Formel (Ia) substituiert ist,
mit wässrigem Formaldehyd, um ein Anfangsreaktionsmedium zu bilden, und Erhitzen des genannten Anfangsreaktionsmediums während einer Zeit von 20 Minuten bis 2 Stunden, insbesondere von ungefähr 30 Minuten bis ungefähr 1 Stunde, wobei gleichzeitig gegebenenfalls das Wasser, das in dem Anfangsreaktionsmedium vorhanden ist, sowie jenes, das gebildet wird, eliminiert wird,
b. Fortsetzen des Erhitzens des genannten Reaktionsmediums während 1 bis 3 Stunden, wobei gleichzeitig das Wasser, das in dem Reaktionsmedium vorhanden ist, sowie jenes, das gebildet wird, eliminiert wird, um ein Reaktionsmedium zu erhalten, das einen festen Vorläufer in der Form eines brüchigen harten Harzes enthält,
c. gegebenenfalls:

o Erhitzen des genannten Reaktionsmediums oder des genannten festen Vorläufers mit Hilfe von Wärmeübertragungsmitteln in der Form eines Ofens während 3 bis 24 Stunden,
o oder Zusetzen, zu dem genannten Reaktionsmedium oder zu dem genannten festen Vorläufer in der Form eines brüchigen harten Harzes, einer Flüssigkeit, in welcher der genannte feste Vorläufer in der

Form eines brüchigen harten Harzes sehr wenig löslich oder vollständig unlöslich ist, insbesondere in Toluol, Xylol, einem linearen oder verzweigten Alkan mit einem Siedepunkt von mehr als 50 °C, insbesondere mehr als 100 °C, insbesondere Octan oder einem Silikonöl, um ein Reaktionsmedium zu erhalten, das einen Feststoff in der Form eines brüchigen harten Harzes enthält, und Erhitzen des genannten Reaktionsmediums, das einen Feststoff in der Form eines brüchigen harten Harzes enthält, während 3 bis 24 Stunden, mit oder ohne Rühren,
um eine Mischung (b) zu erhalten, umfassend das genannte große *p*-(R)-Calixaren oder die genannte Mischung (a) von mindestens zwei der genannten großen *p*-(R)-Calixarene, wobei die genannte Mischung (b) ferner eine Mischung (c) von riesigen *p*-(R)-Calixarenen umfasst, deren Größe größer ist als 20,

d. gegebenenfalls, nach der Neutralisation der Base, mindestens eine Wäsche der genannten Mischung (b) mit einem polaren Lösungsmittel, insbesondere Methanol oder Acetonitril, um die genannte Mischung (b) in neutralisierter Form zu erhalten,
e. Reinigen der genannten gegebenenfalls neutralisierten Mischung (b), um die genannte Mischung (c) von riesigen *p*-(R)-Calixarenen zu eliminieren, und um das genannte große *p*-(R)-Calixaren oder die genannte Mischung (a) von mindestens zwei der genannten großen *p*-(R)-Calixarene ohne die genannte Mischung (c) der riesigen *p*-(R)-Calixarene zu erhalten,
f. gegebenenfalls Reinigen der Mischung (a) durch GPC, indem die verschiedenen Fraktionen in Abhängigkeit von ihrer Elutionszeit getrennt werden, um das genannte große *p*-(R)-Calixaren zu erhalten.

**6.** Verfahren nach einem der Ansprüche 1 bis 5, umfassend die folgenden Schritte:

a. Inberührungbringen mindestens einer Base, die insbesondere aus Bariumhydroxid, Rubidiumhydroxid, Lithiumhydroxid, Cesiumhydroxid, Kaliumchlorid oder Natriumcarbonat ausgewählt ist, mit mindestens einem Phenol, das in Stellung 4 der Formel (I), insbesondere der Formel (Ia) substituiert ist,
mit wässrigem Formaldehyd, um ein Anfangsreaktionsmedium zu bilden, und Erhitzen des genannten Anfangsreaktionsmediums während einer Zeit von 20 Minuten bis 2 Stunden, insbesondere von ungefähr 30 Minuten bis ungefähr 1 Stunde, wobei gleichzeitig gegebenenfalls das Wasser, das in dem Anfangsreaktionsmedium vorhanden ist, sowie jenes, das gebildet wird, eliminiert wird,
b. Fortsetzen des Erhitzens des genannten Reaktionsmediums während 1 bis 3 Stunden, wobei gleichzeitig das Wasser, das in dem Reaktionsmedium vorhanden ist, sowie jenes, das gebildet wird, eliminiert wird, um ein Reaktionsmedium zu erhalten, das einen festen Vorläufer in der Form eines brüchigen harten Harzes enthält,
c. Erhitzen des genannten Reaktionsmediums oder des genannten festen Vorläufers mit Hilfe von Wärmeübertragungsmitteln in der Form eines Ofens während 3 bis 24 Stunden,
oder Zusetzen, zu dem genannten Reaktionsmedium oder zu dem genannten festen Vorläufer in der Form eines brüchigen harten Harzes, einer Flüssigkeit, in welcher der genannte feste Vorläufer in der Form eines brüchigen harten Harzes sehr wenig löslich oder vollständig unlöslich ist, insbesondere in Toluol, Xylol, einem linearen oder verzweigten Alkan mit einem Siedepunkt von mehr als 50 °C, insbesondere mehr als 100 °C, insbesondere Octan oder einem Silikonöl, um ein Reaktionsmedium zu erhalten, das einen Feststoff in der Form eines brüchigen harten Harzes enthält, und Erhitzen des genannten Reaktionsmediums, das einen Feststoff in der Form eines brüchigen harten Harzes enthält, während 3 bis 24 Stunden, mit oder ohne Rühren,
um eine Mischung (b) zu erhalten, umfassend das genannte große *p*-(R)-Calixaren oder die genannte Mischung (a) von mindestens zwei der genannten großen *p*-(R)-Calixarene, wobei die genannte Mischung (b) ferner eine Mischung (c) von riesigen *p*-(R)-Calixarenen umfasst, deren Größe größer ist als 20,
d. gegebenenfalls, nach der Neutralisation der Base, mindestens eine Wäsche der genannten Mischung (b) mit einem polaren Lösungsmittel, insbesondere Methanol oder Acetonitril, um die genannte Mischung (b) in neutralisierter Form zu erhalten,
e. Reinigen der genannten gegebenenfalls neutralisierten Mischung (b), um die genannte Mischung (c) von riesigen *p*-(R)-Calixarenen zu eliminieren, und um das genannte große *p*-(R)-Calixaren oder die genannte Mischung (a) von mindestens zwei der genannten großen *p*-(R)-Calixarene ohne die genannte Mischung (c) der riesigen *p*-(R)-Calixarene zu erhalten,
f. gegebenenfalls Reinigen der Mischung (a) durch GPC, indem die verschiedenen Fraktionen in Abhängigkeit von ihrer Elutionszeit getrennt werden, um das genannte große *p*-(R)-Calixaren zu erhalten.

**7.** Verfahren nach einem der Ansprüche 1 bis 3, wobei das Wasser, das in dem Anfangsreaktionsmedium vorhanden ist, sowie jenes, das während der Reaktion gebildet wird, in dem Reaktionsmedium während des genannten Erhitzens beibehalten wird, und ein fester Vorläufer in der Form eines Niederschlags, der gegebenenfalls isoliert wird, erhalten

wird,

* wobei insbesondere der genannte feste Vorläufer in der Form eines Niederschlags aus dem genannten Reaktionsmedium isoliert wird und keiner ergänzenden thermischen Behandlung unterzogen wird,
* wobei das genannte Verfahren insbesondere die folgenden Schritte umfasst:

a. Inberührungbringen mindestens einer Base, die insbesondere aus Bariumhydroxid, Rubidiumhydroxid, Lithiumhydroxid, Cesiumhydroxid, Kaliumchlorid oder Natriumcarbonat ausgewählt ist, mit mindestens einem Phenol, das in Stellung 4 der Formel (I), insbesondere der Formel (Ia) substituiert ist,
mit wässrigem Formaldehyd, um ein Anfangsreaktionsmedium zu bilden, und Erhitzen des genannten Anfangsreaktionsmediums während einer Zeit von 20 Minuten bis 5 Stunden, insbesondere von ungefähr 30 Minuten bis ungefähr 2 Stunden, ohne Eliminierung des Wassers, das in dem Anfangsreaktionsmedium vorhanden ist, sowie jenes, das gebildet wird, um ein Reaktionsmedium zu erhalten, das einen festen Vorläufer in der Form eines Niederschlags enthält,
b. gegebenenfalls Zusetzen, zu dem genannten Reaktionsmedium oder zu dem genannten festen Vorläufer in der Form eines Niederschlags, einer Flüssigkeit, in welcher der genannte feste Vorläufer in der Form eines Niederschlags sehr wenig löslich oder vollständig unlöslich ist, insbesondere in Toluol, Xylol, einem linearen oder verzweigten Alkan mit einem Siedepunkt von mehr als 50 °C, insbesondere mehr als 100 °C, insbesondere Octan oder einem Silikonöl, um ein Reaktionsmedium zu erhalten, das einen Feststoff in der Form eines Niederschlags enthält, und Erhitzen des genannten Reaktionsmediums, das einen Feststoff in der Form eines Niederschlags enthält, während 3 bis 24 Stunden, mit oder ohne Rühren,
um eine Mischung (b) zu erhalten, umfassend das genannte große *p*-(R)-Calixaren oder die genannte Mischung (a) von mindestens zwei der genannten großen *p*-(R)-Calixarene, wobei die genannte Mischung (b) ferner eine Mischung (c) von riesigen *p*-(R)-Calixarenen umfasst, deren Größe größer ist als 20,
c. gegebenenfalls, nach der Neutralisation der Base, mindestens eine Wäsche der genannten Mischung (b) mit einem polaren Lösungsmittel, insbesondere Methanol oder Acetonitril, um die genannte Mischung (b) in neutralisierter Form zu erhalten,
d. Reinigen der genannten gegebenenfalls neutralisierten Mischung (b), um die genannte Mischung (c) von riesigen *p*-(R)-Calixarenen zu eliminieren, und um das genannte große *p*-(R)-Calixaren oder die genannte Mischung (a) von mindestens zwei der genannten großen *p*-(R)-Calixarene ohne die genannte Mischung (c) der riesigen *p*-(R)-Calixarene zu erhalten,
e. gegebenenfalls Reinigen der Mischung (a) durch GPC, indem die verschiedenen Fraktionen in Abhängigkeit von ihrer Elutionszeit getrennt werden, um das genannte große *p*-(R)-Calixaren zu erhalten.

**8.** Verfahren nach Anspruch 7, wobei das Wasser, das in dem Anfangsreaktionsmedium vorhanden ist, sowie jenes, das während der Reaktion gebildet wird, in dem genannten Reaktionsmedium während des genannten Erhitzens beibehalten wird, und ein fester Vorläufer in der Form eines Niederschlags, der gegebenenfalls isoliert wird, erhalten wird,

* wobei insbesondere der genannte feste Vorläufer in der Form eines Niederschlags aus dem genannten Reaktionsmedium isoliert wird und keiner ergänzenden thermischen Behandlung unterzogen wird,
* wobei das genannte Verfahren insbesondere die folgenden Schritte umfasst:

a. Inberührungbringen mindestens einer Base, die insbesondere aus Bariumhydroxid, Rubidiumhydroxid, Lithiumhydroxid, Cesiumhydroxid, Kaliumchlorid oder Natriumcarbonat ausgewählt ist, mit mindestens einem Phenol, das in Stellung 4 der Formel (I), insbesondere der Formel (Ia) substituiert ist,
mit wässrigem Formaldehyd, um ein Anfangsreaktionsmedium zu bilden, und Erhitzen des genannten Anfangsreaktionsmediums während einer Zeit von 20 Minuten bis 5 Stunden, insbesondere von ungefähr 30 Minuten bis ungefähr 2 Stunden, ohne Eliminierung des Wassers, das in dem Anfangsreaktionsmedium vorhanden ist, sowie jenes, das gebildet wird, um ein Reaktionsmedium zu erhalten, das einen festen Vorläufer in der Form eines Niederschlags enthält,
b. gegebenenfalls Zusetzen, zu dem genannten Reaktionsmedium oder zu dem genannten festen Vorläufer in der Form eines Niederschlags, einer Flüssigkeit, in welcher der genannte feste Vorläufer in der Form eines Niederschlags sehr wenig löslich oder vollständig unlöslich ist, insbesondere in Toluol, Xylol, einem linearen oder verzweigten Alkan mit einem Siedepunkt von mehr als 50 °C, insbesondere mehr als 100 °C, insbesondere Octan oder einem Silikonöl, um ein Reaktionsmedium zu erhalten, das einen Feststoff in der Form eines Niederschlags enthält, und Erhitzen des genannten Reaktionsmediums, das einen Feststoff in der Form eines Niederschlags enthält, während 3 bis 24 Stunden, mit oder ohne Rühren,

um eine Mischung (b) zu erhalten, umfassend das genannte große *p*-(R)-Calixaren oder die genannte Mischung (a) von mindestens zwei der genannten großen *p*-(R)-Calixarene, wobei die genannte Mischung (b) ferner eine Mischung (c) von riesigen *p*-(R)-Calixarenen umfasst, deren Größe größer ist als 20,
c. gegebenenfalls, nach der Neutralisation der Base, mindestens eine Wäsche der genannten Mischung (b) mit einem polaren Lösungsmittel, insbesondere Methanol oder Acetonitril, um die genannte Mischung (b) in neutralisierter Form zu erhalten,
d. Reinigen der genannten gegebenenfalls neutralisierten Mischung (b), um die genannte Mischung (c) von riesigen *p*-(R)-Calixarenen zu eliminieren, und um das genannte große *p*-(R)-Calixaren oder die genannte Mischung (a) von mindestens zwei der genannten großen *p*-(R)-Calixarene ohne die genannte Mischung (c) der riesigen *p*-(R)-Calixarene zu erhalten,
e. gegebenenfalls Reinigen der Mischung (a) durch GPC, indem die verschiedenen Fraktionen in Abhängigkeit von ihrer Elutionszeit getrennt werden, um das genannte große *p*-(R)-Calixaren zu erhalten.

**9.** Verfahren nach einem der Ansprüche 1 bis 8, wobei die genannte Reinigung der genannten Mischung (b) einen Umkristallisationsschritt der genannten Mischung (b) umfasst, um am Ende des Umkristallisationsschritts einen Niederschlag und ein Filtrat zu erhalten, wobei das genannte Filtrat das genannte große *p*-(R)-Calixaren oder die genannte Mischung (a) von mindestens zwei der genannten großen *p*-(R)-Calixarene ohne die genannte Mischung (c) der riesigen *p*-(R)-Calixarene umfasst, und der Niederschlag die genannte Mischung (c) von riesigen *p*-(R)-Calixarenen umfasst, deren Größe größer ist als 20.

**10.** Verfahren nach einem der Ansprüche 1 bis 8, wobei die genannte Reinigung der Mischung (b) einen Umkristallisationsschritt mit zwei Lösungsmitteln der genannten Mischung (b) umfasst, um, nach dem Zusatz des zweiten Lösungsmittels, einen Niederschlag und ein Filtrat zu erhalten, wobei das genannte Filtrat das genannte große *p*-(R)-Calixaren oder die genannte Mischung (a) von mindestens zwei der genannten großen *p*-(R)-Calixarene und die Mischung (c) der riesigen *p*-(R)-Calixarene umfasst, und der Niederschlag hauptsächlich Calix[8]aren umfasst.

**11.** Verfahren nach einem der Ansprüche 1 bis 8, wobei die genannte Reinigung der genannten Mischung (b) die folgenden Schritte umfasst:

- Lösen der genannten Mischung (b) in dem Minimum eines ersten Lösungsmittels, heiß, um eine heiße Lösung der genannten Mischung (b) zu erhalten,
- Zusetzen, zu der heißen Lösung der genannten Mischung (b), eines zweiten Lösungsmittels bis zum Auftreten eines Niederschlags und sofortiges Filtrieren, um ein Filtrat zu erhalten, das die großen Calixarene und die riesigen Calixarene enthält,
- nach Abkühlen des zuvor erhaltenen Filtrats, Filtrieren des genannten Niederschlags, um zu erhalten:

  ◦ ein Filtrat, umfassend das genannte große *p*-(R)-Calixaren oder die genannte Mischung (a) von mindestens zwei der genannten großen *p*-(R)-Calixarene ohne die genannte Mischung (c) von riesigen *p*-(R)-Calixarenen, und
  ◦ den isolierten Niederschlag, umfassend die genannte Mischung (c) von riesigen *p*-(R)-Calixarenen, deren Größe größer ist als 20.

**12.** Verfahren nach einem der Ansprüche 1 bis 8, wobei die genannte Reinigung der genannten Mischung (b) die folgenden Schritte umfasst:

- Lösen der genannten Mischung (b) in dem Minimum eines ersten Lösungsmittels, heiß, um eine heiße Lösung der genannten Mischung (b) zu erhalten,
- Zusetzen, zu der heißen Lösung der genannten Mischung (b), eines zweiten Lösungsmittels bis zum Auftreten eines ersten Niederschlags,
- Heißfiltrieren des genannten ersten Niederschlags, um ein Filtrat zu erhalten,
- Abkühlen des Filtrats, um die Bildung eines zweiten Niederschlags zu erhalten,
- Filtrieren des genannten zweiten Niederschlags, um zu erhalten:

  o ein Filtrat, umfassend das genannte große *p*-(R)-Calixaren oder die genannte Mischung (a) von mindestens zwei der genannten großen *p*-(R)-Calixarene ohne die genannte Mischung (c) von riesigen *p*-(R)-Calixarenen, und
  ◦ den isolierten Niederschlag, umfassend die genannte Mischung (c) von riesigen *p*-(R)-Calixarenen, deren Größe größer ist als 20.

**13.** Verfahren nach einem der Ansprüche 1 bis 8, wobei die genannte Reinigung der genannten Mischung (b) die folgenden Schritte umfasst:

- Lösen der genannten Mischung (b) in dem Minimum von DMSO bei einer Temperatur von ungefähr 80 °C bis ungefähr 160 °C, insbesondere von ungefähr 100 °C bis ungefähr 140 °C, mehr im Einzelnen bei einer Temperatur von ungefähr 120 °C , um eine heiße Lösung der genannten Mischung (b) zu erhalten,
- Zusetzen, zu der heißen Lösung der genannten Mischung (b), eines zweiten polaren Lösungsmittels, insbesondere Aceton, bis zum Auftreten eines ersten Niederschlags,
- Heißfiltrieren des genannten ersten Niederschlags, um ein Filtrat zu erhalten,
- Abkühlen des Filtrats auf ungefähr 0 °C, um die Bildung eines zweiten Niederschlags zu erhalten,
- Filtrieren des genannten zweiten Niederschlags, um zu erhalten:

  ◦ ein Filtrat, umfassend das genannte große *p*-(R)-Calixaren oder die genannte Mischung (a) von mindestens zwei der genannten großen *p*-(R)-Calixarene ohne die genannte Mischung (c) von riesigen *p*-(R)-Calixarenen, und
  ◦ den isolierten Niederschlag, umfassend die genannte Mischung (c) von riesigen *p*-(R)-Calixarenen, deren Größe größer ist als 20,

- Konzentrieren des genannten Filtrats, um das genannte große *p*-(R)-Calixaren oder die genannte Mischung (a) von mindestens zwei der genannten großen *p*-(R)-Calixarene ohne die genannte Mischung (c) von riesigen *p*-(R)-Calixarenen zu erhalten.

**14.** Verfahren nach einem der Ansprüche 1 bis 8, wobei die genannte Reinigung der genannten Mischung (b) die folgenden Schritte umfasst:

- Lösen der genannten Mischung (b) in dem Minimum von DMSO bei einer Temperatur von ungefähr 80 °C bis ungefähr 160 °C, insbesondere von ungefähr 100 °C bis ungefähr 140 °C, mehr im Einzelnen bei einer Temperatur von ungefähr 120 °C , um eine heiße Lösung der Mischung (b) zu erhalten,
- Zusetzen, zu der heißen Lösung der genannten Mischung (b), eines zweiten polaren Lösungsmittels, insbesondere Aceton, bis zum Auftreten eines ersten Niederschlags,
- Heißfiltrieren des genannten ersten Niederschlags, um ein Filtrat zu erhalten,
- Abkühlen des Filtrats auf ungefähr 0 °C, um die Bildung eines zweiten Niederschlags zu erhalten,
- Filtrieren des genannten zweiten Niederschlags, um zu erhalten:

  ◦ ein Filtrat, umfassend das genannte große *p*-(R)-Calixaren oder die genannte Mischung (a) von mindestens zwei der genannten großen *p*-(R)-Calixarene ohne die genannte Mischung (c) von riesigen *p*-(R)-Calixarenen, und
  ◦ den isolierten Niederschlag, umfassend die genannte Mischung (c) von riesigen *p*-(R)-Calixarenen, deren Größe größer ist als 20,

- Konzentrieren des genannten Filtrats, um einen Rückstand ohne Aceton zu erhalten,
- Zusetzen, zu dem genannten Rückstand, eines polaren Lösungsmittels, insbesondere Ethanol, vor allem bei Umgebungstemperatur, um eine Lösung des genannten Rückstands in dem genannten polaren Lösungsmittel zu erhalten,
- Abkühlen der genannten Lösung des Rückstands, insbesondere auf ungefähr 0 °C, um eine Suspension zu erhalten, wobei die genannte Suspension filtriert wird, um ein Filtrat zu erhalten, wobei das genannte Filtrat konzentriert wird, um das große *p*-(R)-Calixaren oder die genannte Mischung (a) von mindestens zwei der genannten großen *p*-(R)-Calixarene ohne die genannteMischung (c) von riesigen *p*-(R)-Calixarenen zu erhalten.

## Claims

**1.** Process for the preparation of:

- a large *p*-(R)calixarene selected from a *p*-(R)calix[9]arene, a *p*-(R)calix[10]arene, a *p*-(R)calix[11]arene, a *p*-(R)calix[12]arene, a *p*-(R)calix[13]arene, a *p*-(R)calix[14]arene, a *p*-(R)calix[15]arene, a *p*-(R)calix[16]arene, a *p*-(R)calix[17]arene, a *p*-(R)calix[18]arene, a *p*-(R)calix[19]arene, a *p*-(R)calix[20]arene, or
- a mixture of at least two of said large *p*-(R)calixarenes in which said at least two large *p*-(R)calixarenes are

present in said mixture each at a level of at least 5 mol.%,

comprising a stage of bringing at least one base, in particular selected from barium hydroxide, rubidium hydroxide, lithium hydroxide, caesium hydroxide, potassium hydroxide or sodium hydroxide, into contact with at least one phenol substituted in position 4 of the following formula (I):

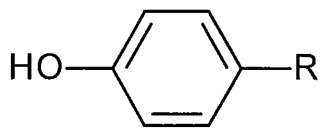

(I)

in which R is selected from:

- a benzyl group optionally substituted in the ortho and/or para and/or meta position with one or more substituents selected from a halogen, SR' and OR', R' representing a linear or branched $C_1$-$C_{20}$ alkyl chain, a $C_3$ to $C_{20}$ cycloalkyl group, $NR_aR_b$, $PR_aR_b$, $P(O)R_aR_b$, $P(O)OR_aOR_b$, $R_a$ and $R_b$ representing, independently of one another, a linear or branched $C_1$-$C_{20}$ alkyl,
- a linear or branched $C_1$-$C_{20}$ alkyl group, with the exclusion of the *t*-butyl group,
- a benzyloxy group optionally substituted in the ortho and/or para and/or meta position with one or more substituents selected from a halogen, SR' and OR', R' representing a linear or branched $C_1$-$C_{20}$ alkyl chain, a $C_3$ to $C_{20}$ cycloalkyl group, $NR_aR_b$, $PR_aR_b$, $P(O)R_aR_b$, $P(O)OR_aOR_b$, $R_a$ and $R_b$ representing, independently of one another, a linear or branched $C_1$-$C_{20}$ alkyl,
- a linear or branched $C_1$-$C_{20}$ alkyloxy group,
- a linear or branched 1 to 10 O-PEG group, the -OH end group of which is alkylated with a linear or branched $C_1$-$C_{20}$ alkyl,
- a benzyl thioether group $-S-CH_2-Ph$ optionally substituted in the ortho and/or para and/or meta position with one or more substituents selected from a halogen, SR' and OR', $NR_aR_b$, $PR_aR_b$, $P(O)R_aR_b$, $P(O)OR_aOR_b$, $R_a$ and $R_b$ representing, independently of one another, a linear or branched $C_1$-$C_{20}$ alkyl,
- a linear or branched $C_1$-$C_{20}$ alkyl thioether group, of formula $-S-(C_1-C_{20}\text{-alkyl})$,
- an $-NR_aR_b$ group, $R_a$ and $R_b$ representing, independently of one another, a linear or branched $C_1$-$C_{20}$ alkyl,
- a dibenzylamine group of formula $-N(benzyl)_2$, in which the two benzyl groups are, independently of one another, optionally substituted in the ortho and/or para and/or meta position with one or more substituents selected from a halogen, SR' and OR', R' representing a linear or branched $C_1$-$C_{20}$ alkyl chain, a $C_3$ to $C_{20}$ cycloalkyl group, $NR_aR_b$, $PR_aR_b$, $P(O)R_aR_b$, $P(O)OR_aOR_b$, $R_a$ and $R_b$ representing, independently of one another, a linear or branched $C_1$-$C_{20}$ alkyl,

and a source of aqueous formaldehyde,
in the absence of an organic solvent, thus constituting an initial reaction medium, said base being at a total concentration comprised from approximately 0.01 to 1 equivalent, in particular comprised from approximately 0.1 equivalent to approximately 0.4 equivalent, in particular equal to 0.15 or 0.4 equivalent or 1 equivalent, with respect to said at least one phenol substituted in position 4 of formula (I),
said initial reaction medium being heated to a temperature comprised from 100 to 165°C, in order to obtain a heated reaction medium,
and comprising, optionally, after said heating, a stage of additional heat treatment of said heated reaction medium, in the presence of heat transfer means, in particular an oven or a heated liquid,
in order to obtain a mixture (b), comprising said large *p*-(R)calixarene or said mixture (a) of at least two of said large *p*-(R)calixarenes, said mixture (b) comprising in addition a mixture (c) of giant *p*-(R)calixarenes, the size of which is greater than 20,
said process also comprising, after said heating and optional additional heat treatment, a stage of purification of said mixture (b), so as to remove said mixture (c) of giant *p*-(R)calixarenes, and to obtain said large *p*-(R)calixarene or said mixture (a) of at least two of said large *p*-(R)calixarenes, devoid of said mixture (c) of giant *p*-(R)calixarenes.

**2.** Process according to claim 1, for the preparation of:

- a large *p*-(R)calixarene selected from a *p*-(R)calix[9]arene, a *p*-(R)calix[10]arene, ap-(R)calix[11]arene, a *p*-(R)calix[12]arene, or

- a mixture (a) of at least two of said large *p*-(R)calixarenes in which said at least two large *p*-(R)calixarenes are present in said mixture each at a level of at least 5 mol.%,

3. Process according to claim 1, for the preparation of:

- a large *p*-($R_1$-oxy)calixarene selected from a *p*-($R_1$-oxy)calix[9]arene, a *p*-($R_1$-oxy)calix[10]arene, a *p*-($R_1$-oxy)calix[11]arene, a *p*-($R_1$-oxy)calix[12]arene, a *p*-($R_1$-oxy)calix[13]arene, a *p*-($R_1$-oxy)calix[14]arene, a *p*-($R_1$-oxy)calix[15]arene, a *p*-($R_1$-oxy)calix[16]arene, a *p*-($R_1$-oxy)calix[17]arene, a *p*-($R_1$-oxy)calix[18]arene, a *p*-($R_1$-oxy)calix[19]arene, a *p*-($R_1$-oxy)calix[20]arene, or
- a mixture of at least two of said large *p*-($R_1$-oxy)calixarenes in which said at least two large *p*-($R_1$-oxy)calixarenes are present in said mixture each at a level of at least 5 mol.%, comprising a stage of bringing at least one base, in particular selected from barium hydroxide, rubidium hydroxide, lithium hydroxide, caesium hydroxide, potassium hydroxide or sodium hydroxide, into contact with at least one phenol substituted in position 4 of the following formula (Ia):

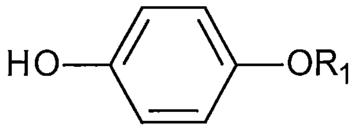

(Ia)

in which $R_1$ is selected from:

- a benzyl group optionally substituted in the ortho and/or para and/or meta position with one or more substituents selected from a halogen, SR' and OR', R' representing a linear or branched $C_1$-$C_{20}$ alkyl chain, a $C_3$ to $C_{20}$ cycloalkyl group, $NR_aR_b$, $PR_aR_b$, $P(O)R_aR_b$, $P(O)OR_aOR_b$, $R_a$ and $R_b$ representing, independently of one another, a linear or branched $C_1$-$C_{20}$ alkyl,
- a linear or branched $C_1$-$C_{20}$ alkyl group,
- a linear or branched PEG 1 to 10, the -OH end group of which is alkylated with a linear or branched $C_1$-$C_{20}$ alkyl,

and a source of aqueous formaldehyde,
in the absence of an organic solvent, thus constituting an initial reaction medium, said base being at a total concentration comprised from approximately 0.01 to 1 equivalent, in particular comprised from approximately 0.1 equivalent to approximately 0.4 equivalent, in particular equal to 0.15 or 0.4 equivalent or 1 equivalent, with respect to said at least one phenol substituted in position 4 of formula (I),
said initial reaction medium being heated to a temperature comprised from 100 to 165°C, in order to obtain a heated reaction medium,
and comprising, optionally, after said heating, a stage of additional heat treatment of said heated reaction medium, in the presence of heat transfer means, in particular an oven or a heated liquid,
in order to obtain a mixture (b), comprising said large *p*-($R_1$-oxy)calixarene or said mixture (a) of at least two of said large *p*-($R_1$-oxy)calixarenes, said mixture (b) comprising in addition a mixture (c) of giant *p*-($R_1$-oxy)calixarenes, the size of which is greater than 20,
said process also comprising, after said heating and optional additional heat treatment, a stage of purification of said mixture (b), so as to remove said mixture (c) of giant *p*-($R_1$-oxy)calixarenes, and to obtain said large *p*-($R_1$-oxy)calixarene or said mixture (a) of at least two of said large *p*-($R_1$-oxy)calixarenes, devoid of said mixture (c) of giant *p*-($R_1$-oxy)calixarenes.

4. Process according to any one of claims 1 to 3, for preparing:

- a large *p*-($R_1$-oxy)calixarene selected from a *p*-($R_1$-oxy)calix[9]arene, a *p*-($R_1$-oxy)calix[10]arene, a *p*-($R_1$-oxy)calix[11]arene, a *p*-($R_1$-oxy)calix[12]arene, or
- a mixture of at least two of said large *p*-($R_1$-oxy)calixarenes in which said at least two large *p*-($R_1$-oxy)calixarenes are present in said mixture each at a level of at least 5 mol.%.

5. Process according to any one of claims 1 to 4, in which the water present in the initial reaction medium, as well as that produced during the reaction, are removed from said reaction medium during said heating and a solid precursor in the form of hard brittle resin, optionally isolated, is obtained,

*in particular in which said solid precursor in the form of hard brittle resin is isolated from the aforesaid reaction medium and not subjected to additional heat treatment,
*in particular in which said solid precursor in the form of hard brittle resin, contained in said reaction medium, is placed in the presence of heat transfer means in the form of an oven, or of a heated liquid in which said solid precursor in the form of hard brittle resin is very slightly soluble or totally insoluble, in particular in toluene, xylene, a linear or branched alkane with a boiling point greater than 50°C, in particular greater than 100°C, in particular octane, or a silicone oil, and is subjected to an additional heat treatment,
*said process comprising in particular the following stages:

a. bringing at least one base, in particular selected from barium hydroxide, rubidium hydroxide, lithium hydroxide, caesium hydroxide, potassium hydroxide or sodium hydroxide, into contact with at least one phenol substituted in position 4 of formula (I), in particular of formula (Ia),
with aqueous formaldehyde, in order to constitute an initial reaction medium, and heating of said initial reaction medium for a time comprised from 20 minutes to 2 hours, in particular from approximately 30 minutes to approximately 1 hour, while optionally removing the water present in the initial reaction medium as well as that formed,
b. continuation of the heating of said reaction medium for 1 to 3 hours, while removing the water present in the reaction medium as well as that formed, in order to obtain a reaction medium containing a solid precursor, in the form of hard brittle resin,
c. optionally:

◦ heating of said reaction medium or said solid precursor using heat transmission means in the form of an oven, for 3 to 24 hours,
◦ or addition to said reaction medium or to said solid precursor in the form of hard brittle resin, of a liquid in which said solid precursor in the form of hard brittle resin is very slightly soluble or totally insoluble, in particular in toluene, xylene, a linear or branched alkane with a boiling point greater than 50°C, in particular greater than 100°C, in particular octane, or a silicone oil, in order to obtain a reaction medium containing a solid in the form of hard brittle resin, and heating of said reaction medium containing a solid in the form of hard brittle resin, for 3 to 24 hours, with or without stirring,
in order to obtain a mixture (b), comprising said large *p*-(R)calixarene or said mixture (a) of at least two of said large *p*-(R)calixarenes, said mixture (b) comprising in addition a mixture (c) of giant *p*-(R)calixarenes, the size of which is greater than 20,

d. optionally, after neutralization of the base, at least one washing of said mixture (b) with a polar solvent, in particular methanol or acetonitrile, in order to obtain said mixture (b) in neutralized form,
e. purification of said mixture (b), optionally neutralized, so as to remove said mixture (c) of giant *p*-(R)calixarenes, and to obtain said large *p*-(R)calixarene or said mixture (a) of at least two of said large *p*-(R)calixarenes, devoid of said mixture (c) of giant *p*-(R)calixarenes,
f. optionally purification of mixture (a) using GPC, by separating different fractions depending on their elution time, in order to obtain said large *p*-(R)calixarene.

**6.** Process according to any one of claims 1 to 5, comprising the following stages:

a. bringing at least one base, in particular selected from barium hydroxide, rubidium hydroxide, lithium hydroxide, caesium hydroxide, potassium hydroxide or sodium hydroxide, into contact with at least one phenol substituted in position 4 of formula (I), in particular of formula (Ia),
with aqueous formaldehyde, in order to constitute an initial reaction medium and heating of said initial reaction medium for a time comprised from 20 minutes to 2 hours, in particular from approximately 30 minutes to approximately 1 hour, while optionally removing the water present in the initial reaction medium as well as that formed,
b. continuation of the heating of said reaction medium for 1 to 3 hours, while optionally removing the water present in the reaction medium as well as that formed, in order to obtain a reaction medium containing a solid precursor, in the form of hard brittle resin,
c. heating of said reaction medium or said solid precursor using heat transmission means in the form of an oven, for 3 to 24 hours,
or addition to said reaction medium or to said solid precursor in the form of hard brittle resin, of a liquid in which said solid precursor in the form of hard brittle resin is very slightly soluble or totally insoluble, in particular in toluene, xylene, a linear or branched alkane with a boiling point greater than 50°C, in particular greater than

100°C, in particular octane, or a silicone oil, in order to obtain a reaction medium containing a solid in the form of hard brittle resin, and heating of said reaction medium containing a solid in the form of hard brittle resin, for 3 to 24 hours, with or without stirring,
in order to obtain a mixture (b), comprising said large *p*-(R)calixarene or said mixture (a) of at least two of said large *p*-(R)calixarenes, said mixture (b) comprising in addition a mixture (c) of giant *p*-(R)calixarenes, the size of which is greater than 20,
d. optionally, after neutralization of the base, at least one washing of said mixture (b) with a polar solvent, in particular methanol or acetonitrile, in order to obtain said mixture (b) in neutralized form,
e. purification of said mixture (b), optionally neutralized, so as to remove said mixture (c) of giant *p*-(R)calixarenes, and to obtain said large *p*-(R)calixarene or said mixture (a) of at least two of said large *p*-(R)calixarenes, devoid of said mixture (c) of giant *p*-(R)calixarenes,
f. optionally, purification of mixture (a) using GPC, by separating different fractions depending on their elution time, in order to obtain said large *p*-(R)calixarene.

**7.** Process according to any one of claims 1 to 3, in which the water present in the initial reaction medium, as well as that produced during the reaction, are retained in the reaction medium during said heating and a solid precursor in the form of precipitate, optionally isolated, is obtained,

*in particular in which said solid precursor in the form of precipitate is isolated from the aforesaid reaction medium and not subjected to additional heat treatment,
*said process comprising in particular the following stages:

a. bringing at least one base, in particular selected from barium hydroxide, rubidium hydroxide, lithium hydroxide, caesium hydroxide, potassium hydroxide or sodium hydroxide, into contact with at least one phenol substituted in position 4 of formula (I), in particular of formula (Ia),
with aqueous formaldehyde, in order to constitute an initial reaction medium and heating of said initial reaction medium for a time comprised from 20 minutes to 5 hours, in particular from approximately 30 minutes to approximately 2 hours, without removing the water present in the initial reaction medium as well as that formed, in order to obtain a reaction medium containing a solid precursor, in the form of precipitate,
b. optionally, addition to said reaction medium or to said solid precursor in the form of precipitate, of a liquid in which said solid precursor in the form of precipitate is very slightly soluble or totally insoluble, in particular in toluene, xylene, a linear or branched alkane with a boiling point greater than 50°C, in particular greater than 100°C, in particular octane, or a silicone oil, in order to obtain a reaction medium containing a solid in the form of precipitate, and heating of said reaction medium containing a solid in the form of precipitate, for 3 to 24 hours, with or without stirring,
in order to obtain a mixture (b), comprising said large *p*-(R)calixarene or said mixture (a) of at least two of said large *p*-(R)calixarenes, said mixture (b) comprising in addition a mixture (c) of giant *p*-(R)calixarenes, the size of which is greater than 20,
c. optionally, after neutralization of the base, at least one washing of said mixture (b) with a polar solvent, in particular methanol or acetonitrile, in order to obtain said mixture (b) in neutralized form,
d. purification of said mixture (b), optionally neutralized, so as to remove said mixture (c) of giant *p*-(R)calixarenes, and to obtain said large *p*-(R)calixarene or said mixture (a) of at least two of said large *p*-(R)calixarenes, devoid of said mixture (c) of giant *p*-(R)calixarenes.
e. optionally, purification of mixture (a) using GPC, by separating different fractions depending on their elution time, in order to obtain said large *p*-(R)calixarene.

**8.** Process according to claim 7, in which the water present in the initial reaction medium, as well as that produced during the reaction, are retained in said reaction medium during said heating and a solid precursor in the form of precipitate, optionally isolated, is obtained,

*in particular in which said solid precursor in the form of precipitate is isolated from the aforesaid reaction medium and not subjected to additional heat treatment,
*said process comprising in particular the following stages:

a. bringing at least one base, in particular selected from barium hydroxide, rubidium hydroxide, lithium hydroxide, caesium hydroxide, potassium hydroxide or sodium hydroxide, into contact with at least one phenol substituted in position 4 of formula (I), in particular of formula (Ia),
with aqueous formaldehyde, in order to constitute an initial reaction medium and heating of said initial

reaction medium for a time comprised from 20 minutes to 5 hours, in particular from approximately 30 minutes to approximately 2 hours, without removing the water present in the initial reaction medium as well as that formed, in order to obtain a reaction medium containing a solid precursor, in the form of precipitate,
b. addition to said reaction medium or to said solid precursor in the form of precipitate of a liquid in which said solid precursor in the form of precipitate is very slightly soluble or totally insoluble, in particular in toluene, xylene, a linear or branched alkane with a boiling point greater than 50°C, in particular greater than 100°C, in particular octane or a silicone oil, in order to obtain a reaction medium containing a solid in the form of precipitate, and heating of said reaction medium containing a solid in the form of precipitate, for 3 to 24 hours, with or without stirring.
in order to obtain a mixture (b), comprising said large *p*-(R)calixarene or said mixture (a) of at least two of said large *p*-(R)calixarenes, said mixture (b) comprising in addition a mixture (c) of giant *p*-(R)calixarenes, the size of which is greater than 20,
c. optionally, after neutralization of the base, at least one washing of said mixture (b) with a polar solvent, in particular methanol or acetonitrile, in order to obtain said mixture (b) in neutralized form,
d. purification of said mixture (b), optionally neutralized, so as to remove said mixture (c) of giant *p*-(R)calixarenes, and to obtain said large *p*-(R)calixarene or said mixture (a) of at least two of said large *p*-(R)calixarenes, devoid of said mixture (c) of giant *p*-(R)calixarenes,
e. optionally purification of mixture (a) using GPC, by separating different fractions depending on their elution time, in order to obtain said large *p*-(R)calixarene.

**9.** Process according to any one of claims 1 to 8, in which said purification of said mixture (b) comprises a stage of recrystallization of said mixture (b) in order to obtain at the end of the recrystallization stage a precipitate and a filtrate, said filtrate comprising said large *p*-(R)calixarene or said mixture (a) of at least two of said large *p*-(R)calixarenes, devoid of said mixture (c) of giant *p*-(R)calixarenes, and the precipitate comprising said mixture (c) of giant *p*-(R)calixarenes, the size of which is greater than 20.

**10.** Process according to any one of claims 1 to 8, in which said purification of said mixture (b) comprises a stage of recrystallization with two solvents of said mixture (b) in order to obtain, after addition of the second solvent, a precipitate and a filtrate, said filtrate comprising said large *p*-(R)calixarene or said mixture (a) of at least two of said large *p*-(R)calixarenes, and mixture (c) of giant *p*-(R)calixarenes, and the precipitate comprising mainly calix[8]arene.

**11.** Process according to any one of claims 1 to 8, in which said purification of said mixture (b) comprises the following stages:

- dissolving said mixture (b) in a minimum amount of a first solvent, hot, in order to obtain a hot solution of said mixture (b),
- adding a second solvent to the hot solution of said mixture (b), until a precipitate appears, and immediate filtration, in order to obtain a filtrate containing the large calixarenes and the giant calixarenes,
- after cooling of the filtrate obtained previously, filtration of said precipitate in order to obtain:

  ◦ a filtrate comprising said large *p*-(R)calixarene or said mixture (a) of at least two of said large *p*-(R)calixarenes, devoid of said mixture (c) of giant *p*-(R)calixarenes, and
  ◦ the isolated precipitate, comprising said mixture (c) of giant *p*-(R)calixarenes, the size of which is greater than 20.

**12.** Process according to any one of claims 1 to 8, in which said purification of said mixture (b) comprises the following stages:

- dissolving said mixture (b) in a minimum amount of a first solvent, hot, in order to obtain a hot solution of said mixture (b),
- adding a second solvent to the hot solution of said mixture (b), until a first precipitate appears,
- hot filtration of said first precipitate in order to obtain a filtrate,
- cooling of the filtrate in order to obtain the formation of a second precipitate,
- filtration of said second precipitate in order to obtain:

  ◦ a filtrate comprising said large *p*-(R)calixarene or said mixture (a) of at least two of said large *p*-(R)calixarenes, devoid of said mixture (c) of giant *p*-(R)calixarenes, and
  ◦ the isolated precipitate, comprising said mixture (c) of giant *p*-(R)calixarenes, the size of which is greater

than 20.

**13.** Process according to any one of claims 1 to 8, in which said purification of said mixture (b) comprises the following stages:

- dissolving said mixture (b) in a minimum amount of DMSO, at a temperature comprised from approximately 80°C to approximately 160°C, in particular from approximately 100°C to approximately 140°C, more particularly at a temperature of approximately 120°C, in order to obtain a hot solution of said mixture (b),
- adding a second, averagely polar solvent, in particular acetone, to the hot solution of said mixture (b), until a first precipitate appears,
- hot filtration of said first precipitate in order to obtain a filtrate,
- cooling of the filtrate to approximately 0°C in order to obtain the formation of a second precipitate,
- filtration of said second precipitate in order to obtain:

    ◦ a filtrate comprising said large *p*-(R)calixarene or said mixture (a) of at least two of said large *p*-(R)calixarenes, devoid of said mixture (c) of giant *p*-(R)calixarenes, and
    ◦ the isolated precipitate, comprising said mixture (c) of giant *p*-(R)calixarenes, the size of which is greater than 20,

- concentration of said filtrate in order to obtain said large *p*-(R)calixarene or said mixture (a) of at least two of said large *p*-(R)calixarenes, devoid of said mixture (c) of giant *p*-(R)calixarenes.

**14.** Process according to any one of claims 1 to 8, in which said purification of said mixture (b) comprises the following stages:

- dissolving said mixture (b) in a minimum amount of DMSO, at a temperature comprised from approximately 80°C to approximately 160°C, in particular from approximately 100°C to approximately 140°C, more particularly at a temperature of approximately 120°C, in order to obtain a hot solution of said mixture (b),
- adding a second, averagely polar solvent, in particular acetone, to the hot solution of said mixture (b), until a first precipitate appears,
- hot filtration of said first precipitate in order to obtain a filtrate,
- cooling of the filtrate to approximately 0°C in order to obtain the formation of a second precipitate,
- filtration of said second precipitate in order to obtain:

    o a filtrate comprising said large *p*-(R)calixarene or said mixture (a) of at least two of said large *p*-(R)calixarenes, devoid of said mixture (c) of giant *p*-(R)calixarenes, and
    ◦ the isolated precipitate, comprising said mixture (c) of giant *p*-(R)calixarenes, the size of which is greater than 20.

- concentration of said filtrate in order to obtain a residue devoid of acetone,
- addition to said residue of a polar solvent, in particular ethanol, in particular at ambient temperature, in order to obtain a solution of said residue in said polar solvent,
- cooling of said solution of said residue, in particular to approximately 0°, in order to obtain a suspension, said suspension being filtered in order to obtain a filtrate, said filtrate being concentrated in order to obtain said large *p*-(R)calixarene or said mixture (a) of at least two of said large *p*-(R)calixarenes, devoid of said mixture (c) of giant *p*-(R)calixarenes.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- CA 2251070
- WO 2013088056 A


### Littérature non-brevet citée dans la description


- **GUTSCHE ; D. GUTSCHE et al.** Calixarenes: An Introduction. The Royal Society of Chemistry, 2008
- **Z. ASFARI ; V. BÖHMER ; J. HARROWFIELD ; J. VICENS.** Calixarenes. Kluwer, 2001
- **T. PATRICK ; P. EGAN.** *J. Org. Chem.,* 1977, vol. 42, 382
- **T. PATRICK ; P. EGAN.** *J. Org. Chem.,* 1978, vol. 43, 4280
- **Z. ASFARI ; J. VICENS.** *Tetrahedron Lett.,* 1988, vol. 29, 2659
- **F. VOCANSON ; M. PERRIN ; R. LAMARTINE.** *J. Inclusion Phenom. Macrocyclic Chem.,* 2001, vol. 39, 127
- **JERRY L. ATWOOD et al.** *Org. Lett.,* 1999, vol. 1, 1523
- **B. DAHWAN et al.** *Macromolec. Chem.,* 1987, vol. 188, 921
- **C. D. GUTSCHE et al.** *Org. Synth.,* 1990, vol. 68, 234
- **C. D. GUTSCHE et al.** *Org. Synth.,* 1990, vol. 68, 238
- **GUTSCHE et al.** *J. Am. Chem. Soc.,* 1999, vol. 121, 4136